# EUROPEAN PATENT SPECIFICATION

(11) **EP 2 668 923 B1**
(45) Date of publication and mention of the grant of the patent: **03.10.2018**
(21) Application number: 13170016.3
(22) Date of filing: 31.05.2013
(51) Int. Cl.: A61B 18/14, A61M 25/00

(54) **Ablation device with drug delivery component**
Ablationsvorrichtung mit Arzneimittelabgabekomponente
Dispositif d'ablation doté d'un composant d'administration de médicament

(30) Priority: 31.05.2012 US 201261653804 P; 12.06.2012 US 201261658577 P; 29.05.2013 US 201313904478
(43) Date of publication of application: 04.12.2013
(73) Proprietor: Covidien LP, Mansfield, MA 02048 (US)
(72) Inventor: Ohri, Rachit, Framingham, MA Massachusetts 01701 (US); Pham, Lan, Nashua, NH New Hampshire 03063 (US); Blaskovich, Phillip D., Salem, MA Massachusetts 01970 (US); Hull, Les, Attleboro, MA Massachusetts 02703 (US); Ayer, Rupal, Boulder, CO Colorado 80301 (US); Wu, Stephen H., Chesterfield, MO Missouri 63017 (US); Herman, Clifford J., Saint Louis, MO Missouri 63119 (US); Nau, William H., Jr., Longmont, CO Colorado 80504 (US); Rossetto, Francesca, Longmont, CO Colorado 80504 (US); Waller, Allison, Blackstone, MA Massachusetts 01504 (US); Huang, Wenxing, 201100 Shanghai (CN); Dicarlo, Paul, Middleboro, MA Massachusetts 02346 (US)
(74) Representative: Soames, Candida Jane

(56) References cited:
- WO-A1-2009/065058
- WO-A2-2006/138719
- US-A- 5 403 311
- US-A1- 2002 120 260
- US-A1- 2006 041 243

## Description

### BACKGROUND

### 1. Technical Field

The present disclosure relates to electrosurgical systems and devices for performing medical procedures. The present disclosure relates to the administration of beneficial agents in general, which include any physiologically, pharmacologically active and/or psychotropic substance(s). More particularly, the present disclosure relates to ablation devices with drug delivery components, ablation needles with drug delivery components, and electrosurgical systems including the same.

### 2. Discussion of Related Art

Electrosurgical instruments have become widely used by surgeons. Electrosurgery involves the application of thermal and/or electrical energy to cut, dissect, ablate, coagulate, cauterize, seal or otherwise treat biological tissue during a surgical procedure. Electrosurgery is typically performed using a handpiece including a surgical instrument (e.g., end effector, ablation probe, or electrode) adapted to transmit energy to a tissue site during electrosurgical procedures, an electrosurgical generator operable to output energy, and a cable assembly operatively connecting the surgical instrument to the generator.

Treatment of certain diseases requires the destruction of malignant tissue growths, e.g., tumors. Electromagnetic radiation can be used to heat and destroy tumor cells. Treatment may involve inserting ablation probes into tissues where cancerous tumors have been identified. Once the probes are positioned, electromagnetic energy is passed through the probes into surrounding tissue.

In the treatment of diseases such as cancer, certain types of tumor cells have been found to denature at elevated temperatures that are slightly lower than temperatures normally injurious to healthy cells. Known treatment methods, such as hyperthermia therapy, heat diseased cells to temperatures above 41° C while maintaining adjacent healthy cells below the temperature at which irreversible cell destruction occurs. These methods involve applying various forms of energy (e.g., electromagnetic, ultrasonic, etc.) to heat, ablate and/or coagulate tissue. Microwave or radio-frequency energy is sometimes utilized to perform these methods. Radio-frequency (RF) and microwave (MW) energy are electromagnetic radiation in the frequency ranges of 3 kilohertz (kHz) to 300 Megahertz (MHz), and 300 MHz to 300 gigahertz (GHz), respectively. Other procedures utilizing electromagnetic radiation to heat tissue also include coagulation, cutting and/or ablation of tissue.

Electrosurgical devices utilizing electromagnetic radiation have been developed for a variety of uses and applications. A number of devices are available that can be used to provide high bursts of energy for short periods of time to achieve cutting and coagulative effects on various tissues. There are a number of different types of apparatus that can be used to perform ablation procedures. Typically, microwave apparatus for use in ablation procedures include a microwave generator that functions as an energy source, and a microwave surgical instrument (e.g., microwave ablation probe) having an antenna assembly for directing the energy to the target tissue. The microwave generator and surgical instrument are typically operatively coupled by a cable assembly having a plurality of conductors for transmitting microwave energy from the generator to the instrument, and for communicating control, feedback and identification signals between the instrument and the generator.

The basic purpose of both monopolar and bipolar electrosurgery is to produce heat to achieve the desired tissue/clinical effect. In monopolar electrosurgery, devices use an instrument with a single, active electrode to deliver energy from an electrosurgical generator to tissue, and a patient return electrode (usually a plate positioned on the patient's thigh or back) as the means to complete the electrical circuit between the electrosurgical generator and the patient. In bipolar electrosurgery, the electrosurgical device includes two electrodes that are located in proximity to one another for the application of current between their surfaces. Bipolar electrosurgical current travels from one electrode, through the intervening tissue to the other electrode to complete the electrical circuit.

The benefits provided by controlled delivery of active agents for the treatment of injury or disease are well recognized in the art and various approaches have been taken to realize the goal of delivering active agents at desired rates over predetermined periods of time. Various different implantable controlled delivery formulations are known in the art, and various different mechanisms have been employed for delivering active agent from implantable formulations at a controlled rate over time.

Medical imaging has become a significant component in the clinical setting and in basic physiology and biology research, e.g., due to enhanced spatial resolution, accuracy and contrast mechanisms that have been made widely available. Medical imaging now incorporates a wide variety of modalities, e.g., computed tomography (CT) and magnetic resonance imaging (MRI), that noninvasively capture the structure and/or function of the human body. Such images are acquired and used in many different ways including medical images for diagnosis, staging and therapeutic management of malignant disease.

Medical image processing, analysis and visualization play an increasingly useful role in disease diagnosis and monitoring as well as, among other things, surgical planning and monitoring of therapeutic procedures. A contrast agent may used for enhancement of the contrast of structures or fluids within the body (or region of interest) in medical imaging to allow visualization and evaluation of lesions seen minimally, if at all, with imaging alone. There is a continuing need for devices capable of dispensing a contrast agent to enhance the visualization of the lesion during the procedure.

Despite advancements in the use of electrosurgical devices for treating biological tissue, there are still concerns for tumor reoccurrence. A continuing need exists for devices capable of dispensing a controlled delivery formulation of a desired active agent, which may help to reduce or eliminate tumor reoccurrence.

The documents US2002/120260, US 2006/041243, US5403311 and WO 2006/138719 disclose medical systems similar to the invention. Further the features of the preamble of claim 1 are disclosed in WO 2009/065058.

### SUMMARY

There is a need for ablation devices capable of dispensing a controlled delivery formulation of a desired active agent. The combination of ablation (e.g., RF ablation and/or microwave ablation) and drug delivery may help to reduce or eliminate tumor reoccurrence. There is a need for an ablation device that is configured to dispense an active agent in a controlled delivery formulation and/or non-active agent (e.g., contrast agent) before, during and/or after ablation, e.g., without the need for further manipulation of the device. A need exists for ablation needles with a drug delivery component.

Electromagnetic energy is generally classified by increasing energy or decreasing wavelength into radio waves, microwaves, infrared, visible light, ultraviolet, X-rays and gamma-rays. As it is used in this description, "ablation procedure" generally refers to any ablation procedure, such as microwave ablation, radio frequency (RF) ablation or microwave ablation-assisted resection.

As it is used in this description, "energy-delivery device" generally refers to any device that can be used to transfer energy from a power generating source, such as a microwave or RF electrosurgical generator, to tissue. For the purposes herein, the term "ablation device" is interchangeable with the term "energy-delivery device." As it is used in this description, "transmission line" generally refers to any transmission medium that can be used for the propagation of signals from one point to another. A transmission line may be, for example, a wire, a two-wire line, a coaxial wire, and/or a waveguide.

For the purposes of this description, the terms "drug," "drug agent," "implantable drug agent," "active agent," "beneficial agent," "therapeutic agent," "therapeutic molecule," and the like are used interchangeably herein, and may include, for example, small molecules, proteins, enzymes, hormones, polynucleotides, nucleoproteins, polysaccharides, glycoproteins, lipoproteins, polypeptides, steroids, analgesics, local anesthetics, antibiotic agents, anti-inflammatory corticosteroids, ocular drugs and synthetic analogs of these species. Some examples of drug agents that may be delivered by devices according to embodiments of the present disclosure are provided later in this description.

There is a need for an implantable formulation that provides pharmacokinetic/ pharmacodynamic (PK/PD) appropriate release rate profile of an active agent without necessarily requiring the need for further manipulation, post implantation or surgical explantation. There is a need for a formulation to facilitate delivery of a wide range of active agents and active agents' formulations, as well as multiple active agents and multiple active agents' formulations, which may also increase the value of each drug-based ablation procedure.

A continuing need exists for systems, devices and methods for controlling and/or monitoring real-time tissue effects to improve patient safety, reduce risk, and/or improve patient outcomes. There is a need for ablation devices capable of dispensing contrast agent to enhance the visualization of the lesion during the treatment procedure.

According to an aspect of the present disclosure, an ablation device is provided. The ablation device includes a handle assembly, an ablation electrode extending from the handle assembly, and one or more delivery needles extending from the handle assembly. The ablation electrode includes an ablation needle. The ablation needle includes a distal end portion including a drug delivery port defined therethrough.

According to an aspect of the present invention, an ablation device is provided. The ablation device includes a handle assembly and an array of ablation electrodes operably associated with the handle assembly. One or more ablation electrodes of the array of ablation electrodes include a recess defined therein. A drug is disposed at least in part within the recess.

The invention itself is limited by the scope of independent claim 1. Preferred embodiments are disclosed in the dependent claims.

According to an aspect of the present disclosure, an ablation system is provided. The ablation system includes a source of electrosurgical energy, a source of coolant fluid, and an ablation electrode assembly operatively connected to the source of electrosurgical energy and fluidly-coupled to the source of coolant fluid. The ablation electrode assembly includes a hub defining a chamber therein and one or more electrically-conductive ablation needles extending from the hub. The ablation system also includes one or more delivery needles extending from the hub. The one or more delivery needles are selectively moveable from a first position, wherein the distal end of the delivery needle is disposed proximal to the distal end portion of the ablation needle, to at least a second position, wherein at least the distal end of the delivery needle is disposed distally beyond the distal end portion of the ablation needle.

### BRIEF DESCRIPTION OF THE DRAWINGS

Objects and features of the presently-disclosed ablation devices with drug delivery (and/or contrast agent) components, ablation needles with drug delivery components, and electrosurgical systems including the same will become apparent to those of ordinary skill in the art when descriptions of various exemplary embodiments thereof are read with reference to the accompanying drawings. The embodiments of the invention are clearly marked as the invention in the following drawings and throughout the description.
FIG. 1A is a schematic diagram of an ablation system including an ablation electrode assembly in accordance with an embodiment of the present disclosure;
FIG. 1B is a schematic diagram of an ablation electrode assembly including the needle assembly of the ablation electrode assembly shown in FIG. 1A in accordance with an embodiment of the present disclosure;
FIG. 2 is a schematic diagram of an ablation system including an energy applicator in accordance with an embodiment of the present disclosure;
FIG. 3 is an enlarged, perspective view of an ablation system including an electrode array in accordance with an embodiment of the present disclosure;
FIG. 4 is an enlarged, perspective view of an ablation system including an electrode array that includes a drug delivery component in accordance with an embodiment of the present disclosure;
FIG. 5A is an enlarged, perspective view of an ablation system including an ablation device that includes ablation electrodes and a delivery needle, shown with the delivery needle disposed in a first configuration, in accordance with an embodiment of the present disclosure;
FIG. 5B is an enlarged, perspective view of the ablation device shown in FIG. 5A shown with the delivery needle disposed in a second configuration in accordance with an embodiment of the present disclosure;
FIG. 6A is an enlarged, perspective view of a portion of an ablation needle, with portions removed, shown with an integral delivery needle disposed in a first configuration in accordance with an embodiment of the present disclosure;
FIG. 6B is an enlarged, perspective view of the ablation needle shown in FIG. 6A shown with the integral delivery needle disposed in a second configuration in accordance with an embodiment of the present disclosure;
FIG. 7A is an enlarged, perspective view of a portion of an ablation needle, with portions removed, shown with an integral delivery needle disposed in a first configuration in accordance with another embodiment of the present disclosure;
FIG. 7B is an enlarged, perspective view of the ablation needle shown in FIG. 7A shown with the integral delivery needle disposed in a second configuration in accordance with an embodiment of the present disclosure;
FIG. 8 is an enlarged, perspective view of a portion of an ablation needle assembly that includes a shaft portion and an interchangeable sleeve member disposed around the shaft portion, the sleeve member including a plurality of drug reservoir divots associated therewith, in accordance with an embodiment of the present disclosure;
FIG. 9 is an enlarged, perspective view of a portion of an ablation needle that includes a shaft portion including a plurality of drug reservoir divots associated therewith in accordance with an embodiment of the present disclosure;
FIG. 10 is an enlarged, perspective view of a portion of an ablation needle assembly that includes an interchangeable sleeve member including a plurality of drug reservoir divots associated therewith in accordance with an embodiment of the present disclosure;
FIG. 11 is an enlarged, perspective view of a portion of an ablation needle assembly that includes an interchangeable sleeve member including a plurality of drug reservoir divots associated therewith and a plurality of apertures defined therethrough in accordance with an embodiment of the present disclosure;
FIG. 12 is an enlarged, perspective view of a portion of an ablation needle assembly that includes an interchangeable sleeve member including a plurality of drug reservoir divots associated therewith and a plurality of elongated apertures defined therethrough in accordance with an embodiment of the present disclosure;
FIG. 13 is an enlarged, perspective view of an RF ablation device including a drug reservoir and a moveable tip portion containing deployable tendrils, shown with the tip portion disposed in a first configuration, in accordance with an embodiment of the present disclosure;
FIG. 14A is an enlarged, perspective view of the RF ablation device shown in FIG. 13 shown with the tip portion disposed in a second configuration, showing the transfer of drugs from the drug reservoir to tissue, in accordance with an embodiment of the present disclosure;
FIG. 14B is an enlarged, perspective view of the RF ablation device shown in FIG. 14A showing deployment of tendrils from the tip portion into tissue in accordance with an embodiment of the present disclosure;
FIG. 15 is a schematic diagram of a distal portion of an ablation device including deployable tendrils in accordance with an embodiment of the present disclosure;
FIG. 16A is an enlarged, perspective view of an ablation device including a shaft portion that includes a moveable member and deployable tendrils, shown with the tendrils disposed in a first configuration, in accordance with an embodiment of the present disclosure;
FIG. 16B is an enlarged, perspective view of the ablation device shown in FIG. 16A, shown with the tendrils disposed in a second configuration, in accordance with an embodiment of the present disclosure;
FIG. 17A is a schematic diagram of a portion of an RF ablation device including a barrel portion configured to contain antenna filaments deployable therefrom and a moveable tip portion, shown with the tip portion disposed in a first configuration, in accordance with an embodiment of the present disclosure;
FIG. 17B is a schematic diagram of the RF ablation device shown in FIG. 16 shown with the tip portion disposed in a second configuration, showing deployment of the antenna filaments from the barrel portion into tissue, in accordance with an embodiment of the present disclosure;
FIG. 18A is a schematic diagram of the RF ablation device shown in FIG. 16 shown with the tip portion disposed in a third configuration, showing the deployed antenna filaments attached in part to the tip portion, in accordance with an embodiment of the present disclosure;
FIG. 18B is a schematic diagram of the RF ablation device shown in FIG. 18A shown with the tip portion disposed in a fourth configuration, showing the antenna filaments separated from the tip portion in accordance with an embodiment of the present disclosure;
FIG. 19 is an enlarged, cross-sectional view of an ablation device that includes an antenna assembly including a porous-metal radiating section suitable for drug delivery in accordance with an embodiment of the present disclosure;
FIG. 20 is an enlarged, perspective view of an ablation device that includes an antenna assembly in accordance with an embodiment of the present disclosure;
FIG. 21 is an enlarged view of the indicated area of detail of FIG. 20 showing a distal portion of the antenna assembly disposed in a first configuration in accordance with an embodiment of the present disclosure;
FIG. 22 is an enlarged view of the indicated area of detail of FIG. 20 showing a distal portion of the antenna assembly disposed in a second configuration in accordance with an embodiment of the present disclosure;
FIG. 23 is an enlarged view of a portion of an ablation needle including drug-delivery tines, the ablation needle is shown disposed in a first configuration within target tissue, in accordance with an embodiment of the present disclosure;
FIG. 24 is an enlarged view of the ablation needle including drug-delivery tines shown in FIG. 23 shown with the ablation needle disposed in a second configuration within target tissue in accordance with an embodiment of the present disclosure;
FIG. 25 is an enlarged view of the ablation needle including drug-delivery tines shown in FIG. 23 shown with the ablation needle disposed in a third configuration within target tissue in accordance with an embodiment of the present disclosure;
FIG. 26 is an enlarged view of a portion of an ablation needle including a delivery needle that includes a micro-needle adapted to be slideably moveable within the delivery needle in accordance with an embodiment of the present disclosure;
FIG. 27 is an enlarged view of the delivery needle shown in FIG. 26 shown with the micro-needle shown in a deployed configuration in accordance with an embodiment of the present disclosure;
FIG. 28 is an enlarged view of a portion of a delivery needle, such as the delivery needle of the ablation needle shown in FIG. 7, shown with a micro-needle shown in a deployed configuration in accordance with an embodiment of the present disclosure;
FIG. 29 is an enlarged view of a portion of a delivery needle, such as the delivery needle of the ablation needle shown in FIG. 7, shown with a micro-needle array shown in a deployed configuration in accordance with an embodiment of the present disclosure;
FIG. 30 is an enlarged view of the indicated area of detail of FIG. 29 showing a string of micro-needle elements of the micro-needle array in accordance with an embodiment of the present disclosure;
FIG. 31 is an enlarged view of the string of micro-needle elements shown in FIG. 30 in accordance with another embodiment of the present disclosure;
FIG. 32 is an enlarged view of a string of micro-needle elements of a micro-needle array shown with a drug attached to the micro-needle elements in accordance with an embodiment of the present disclosure;
FIG. 33 is an enlarged view of a configuration of micro-needle elements of a micro-needle array shown with a drug attached to the micro-needle elements in accordance with another embodiment of the present disclosure;
FIG. 34 is an enlarged view of a portion of a delivery needle, such as the delivery needle of the ablation needle shown in FIG. 7, shown with a partially deployed, heat-sensitive material coated, micro-needle array in accordance with an embodiment of the present disclosure;
FIG. 35 is an enlarged view of the indicated area of detail of FIG. 34 showing micro-needle elements of the micro-needle array in accordance with an embodiment of the present disclosure;
FIG. 36A is a enlarged, perspective view of an electrosurgical system including an ablation device that includes an array of ablation electrodes, a delivery needle, and an antenna assembly including a delivery needle in accordance with an embodiment of the present disclosure;
FIG. 36B is an enlarged, perspective view of the indicated area of detail of FIG. 36A in accordance with an embodiment of the present disclosure;
FIG. 37A is a enlarged, perspective view of the electrosurgical system of FIG. 36A shown with the antenna assembly including another embodiment of a delivery needle in accordance with the present disclosure;
FIG. 37B is an enlarged, perspective view of the indicated area of detail of FIG. 37A in accordance with an embodiment of the present disclosure; and
FIG. 38 is an enlarged, perspective view of an ablation device that includes an array of ablation electrodes including a plurality of drug reservoir divots associated therewith in accordance with the invention.

### DETAILED DESCRIPTION

Hereinafter, embodiments of the presently-disclosed ablation devices with drug delivery and/or contrast agent components, ablation needles with drug delivery and/or contrast agent components (e.g., suitable for use with Cool-tip™ RF ablation devices), and electrosurgical systems including the same are described with reference to the accompanying drawings. Like reference numerals may refer to similar or identical elements throughout the description of the figures. As shown in the drawings and as used in this description, and as is traditional when referring to relative positioning on an object, the term "proximal" refers to that portion of the device, or component thereof, closer to the user and the term "distal" refers to that portion of the device, or component thereof, farther from the user.

This description may use the phrases "in an embodiment," "in embodiments," "in some embodiments," or "in other embodiments," which may each refer to one or more of the same or different embodiments in accordance with the present disclosure.

Various embodiments of the present disclosure provide energy-delivery devices including ablation needles with drug delivery and/or contrast agent components. Embodiments may be suitable for use with Cool-tip™ RF ablation devices. Embodiments may be suitable for utilization in open surgical applications. Embodiments may be suitable for utilization with endoscopic and laparoscopic surgical procedures. Embodiments may be implemented using electromagnetic radiation at microwave frequencies, RF frequencies or at other frequencies.

Various embodiments of the present disclosure provide electrosurgical system including an energy delivery device provided with one or more ablation needles with drug delivery (and/or contrast agent) components. Various embodiments of the presently-disclosed electrosurgical systems may be suitable for microwave ablation and for use to pre-coagulate tissue for microwave ablation assisted surgical resection. Various embodiments of the presently-disclosed electrosurgical systems including an ablation device may include any feature or combination of features of the ablation device embodiments disclosed herein.

Various embodiments of the presently-disclosed ablation needle assembly include an elongated body or shaft portion configured to facilitate delivery of one or more drug agents which may be temperature sensitive into tissue, wherein one or more drug agents may be releaseably disposed over at least a portion of body or shaft portion of the ablation needle assembly itself, and/or one or more drug agents may be releaseably disposed over at least a portion of a sleeve member disposed coaxially around the body or shaft portion of the ablation needle assembly.

Drug agents which may be delivered by devices according to embodiments of the present disclosure include drugs which act on the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autacoid systems, the alimentary and excretory systems, the histamine system and the central nervous system. Some examples of implantable drug agents which may be delivered by devices according to embodiments of the present disclosure are provided later in this description.

In accordance with various embodiments, the combination of tissue ablation and drug delivery may help to reduce and/or eliminate tumor reoccurrence. In accordance with various embodiments, the combination ablation devices with drug delivery and/or contrast agent components may help to reduce procedure times and/or eliminate the need for a separate drug-delivery device.

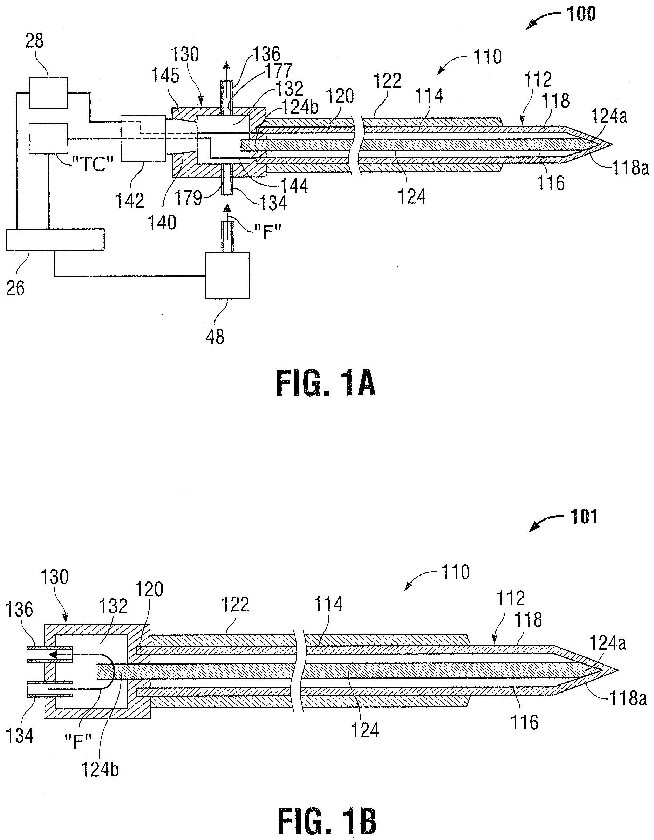

FIG. 1A shows an electrosurgical system (shown generally as 100) in accordance with an embodiment of the present disclosure that includes an ablation electrode assembly 110 and a hub 130 configured to support the ablation electrode assembly 110. Ablation electrode assembly 110 is operatively connected to an electrosurgical power generating source 28, e.g., a microwave or radio frequency (RF) electrosurgical generator. Ablation electrode assembly 110 is disposed in fluid communication with a coolant source 48. Ablation electrode assembly 110 may include additional, fewer, or different components than shown in FIG. 1A, depending upon a particular purpose or to achieve a desired result.

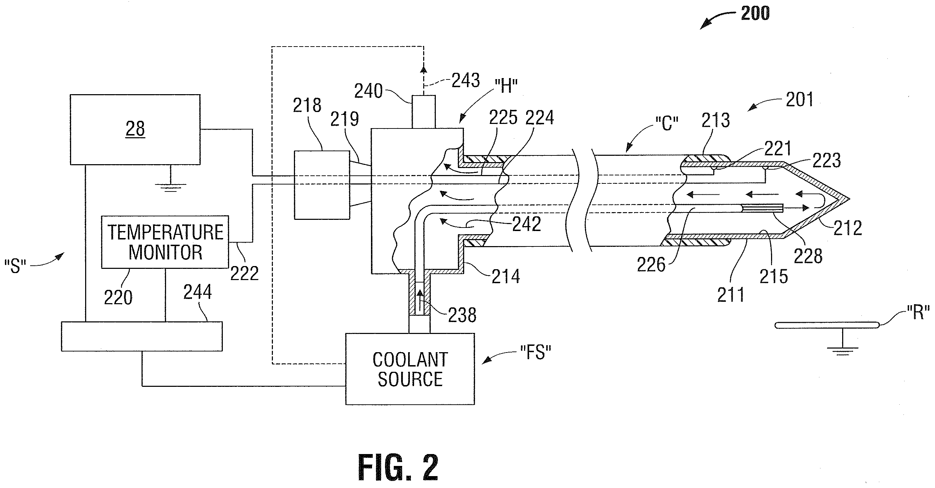

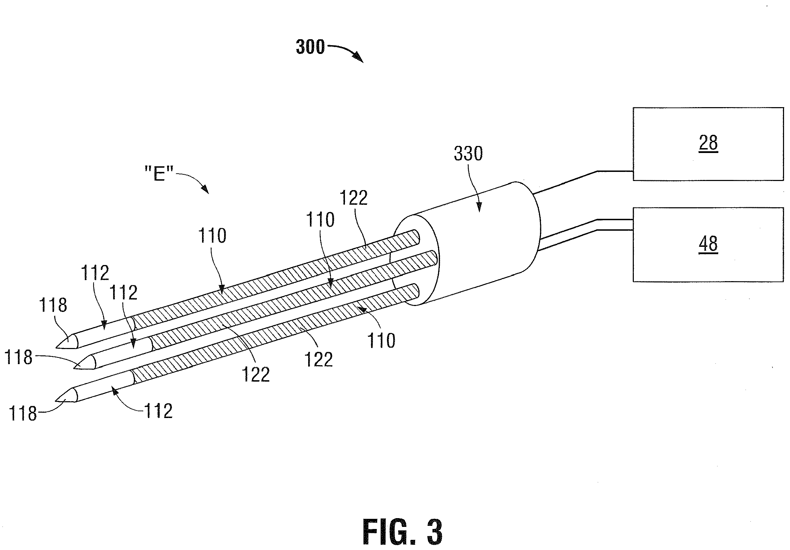

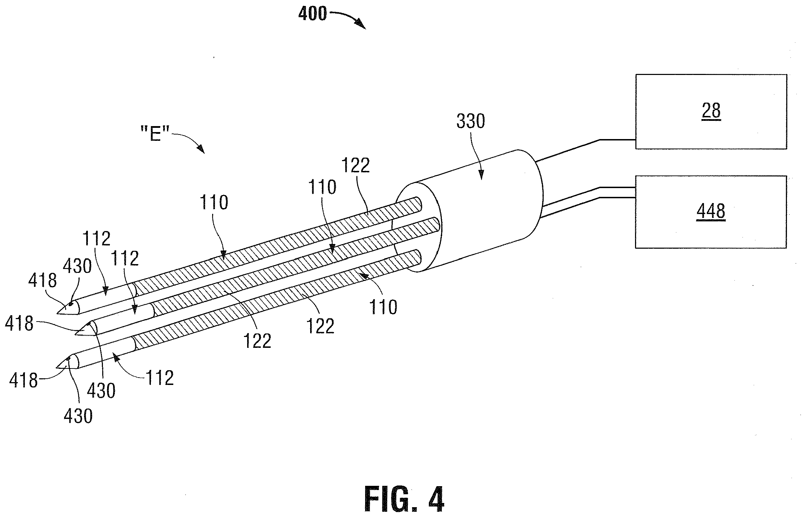

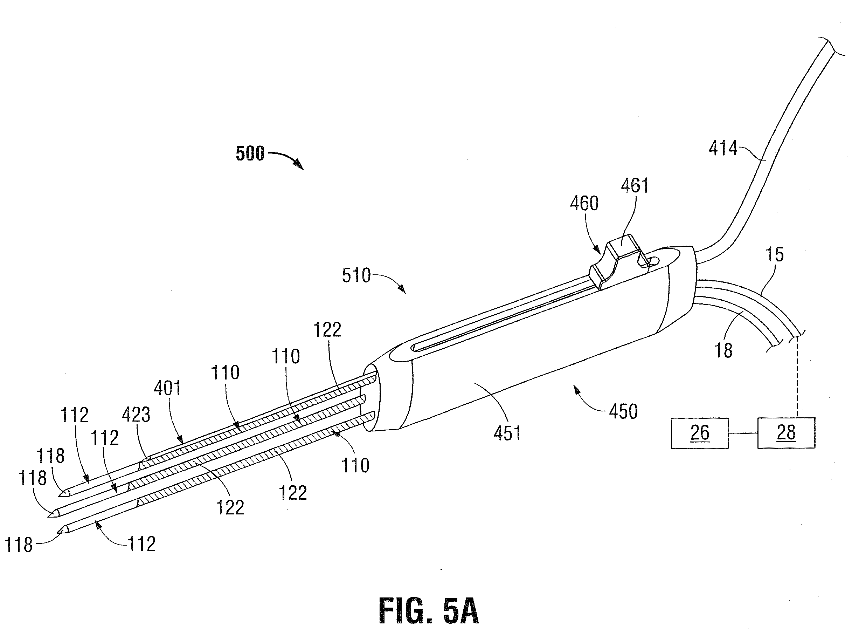

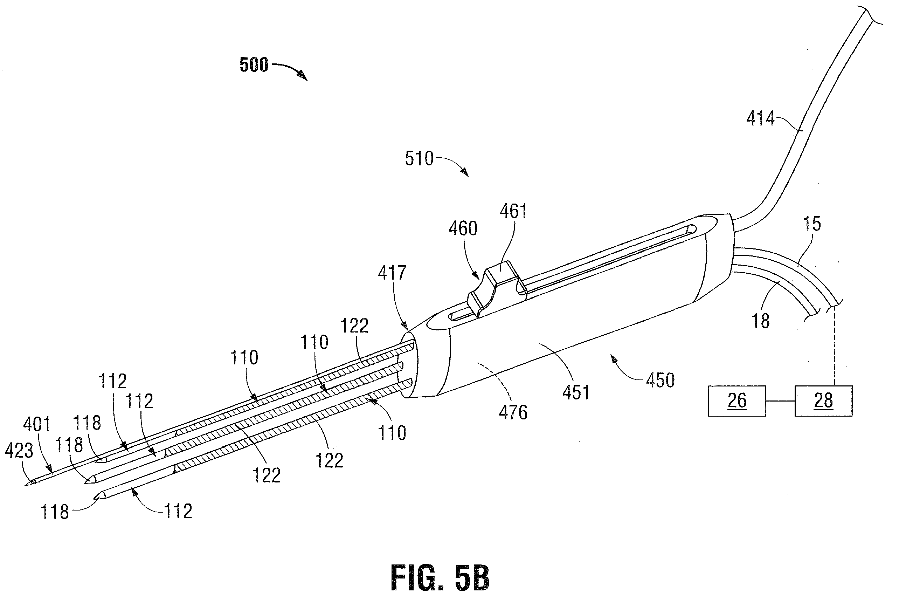

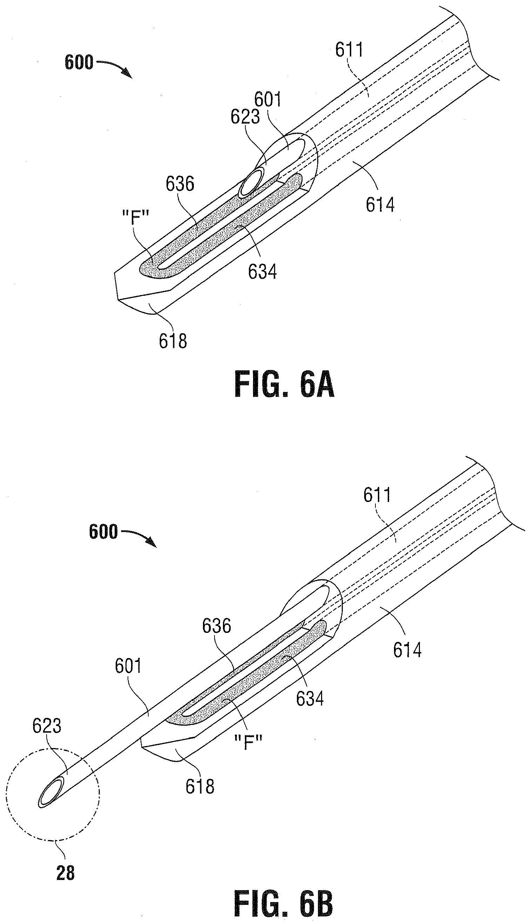

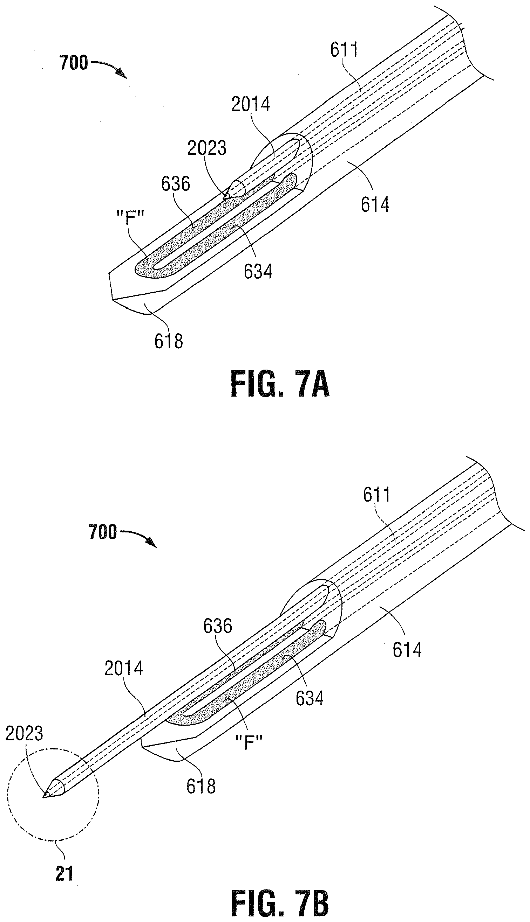

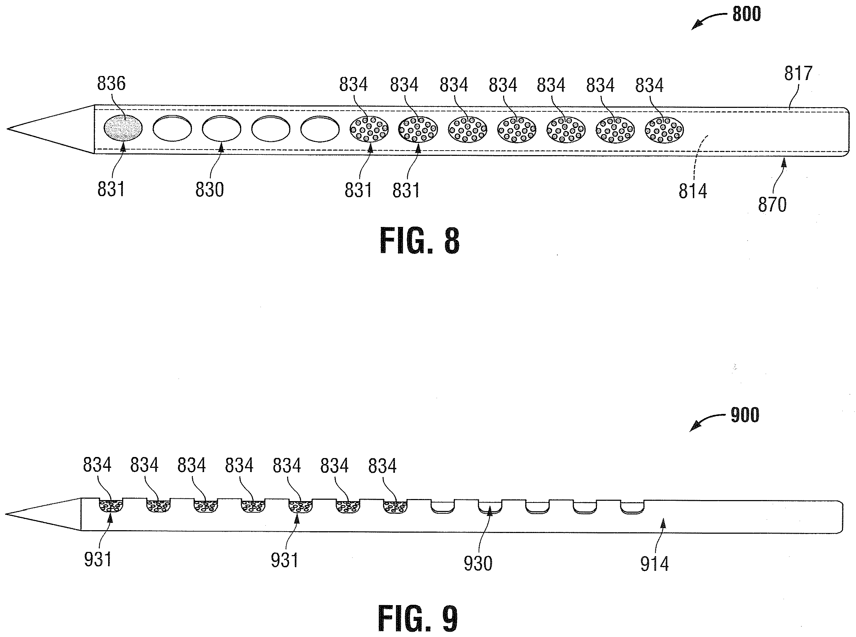

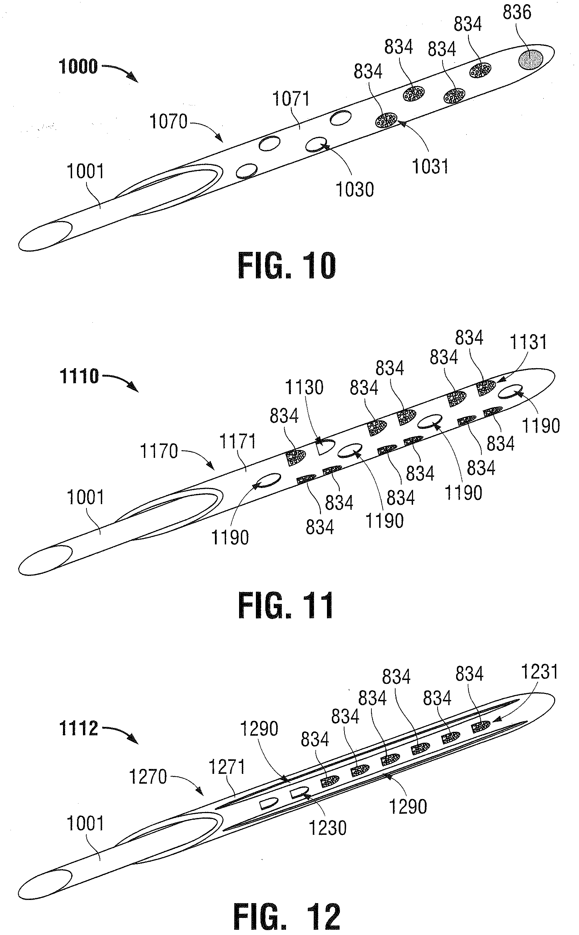

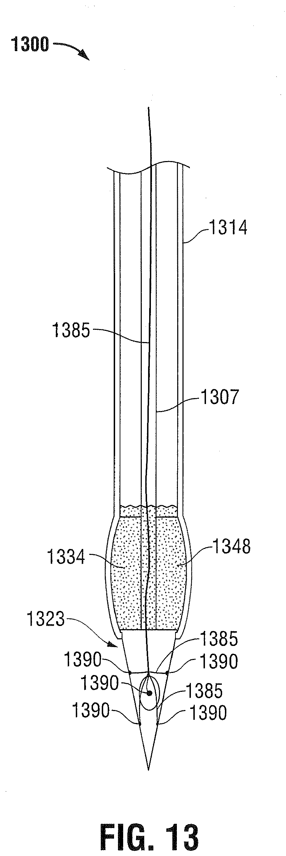

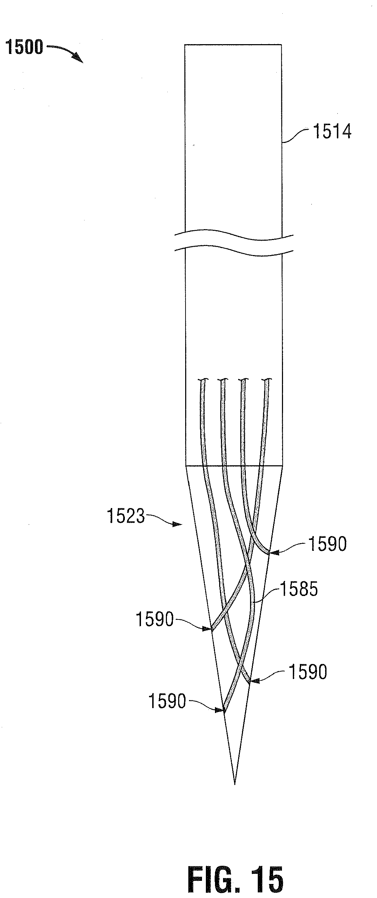

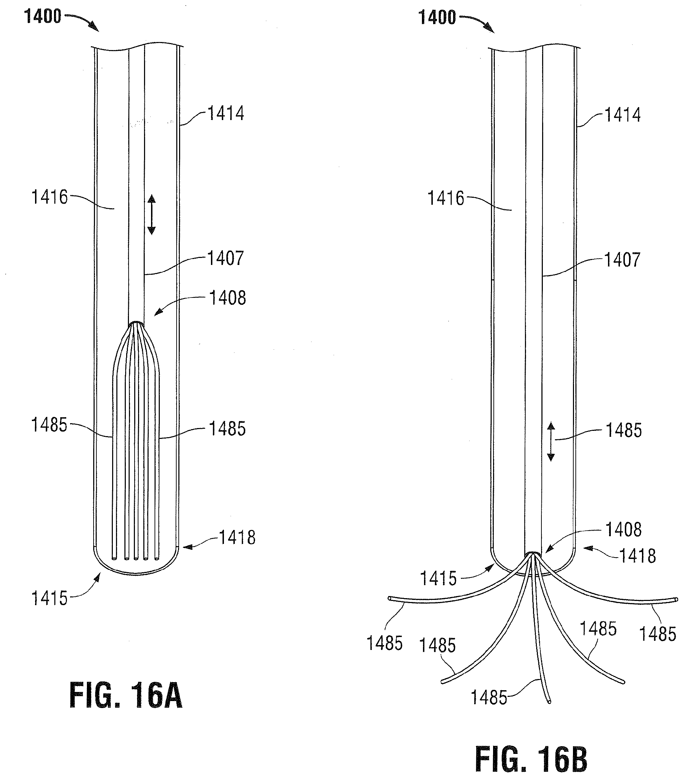

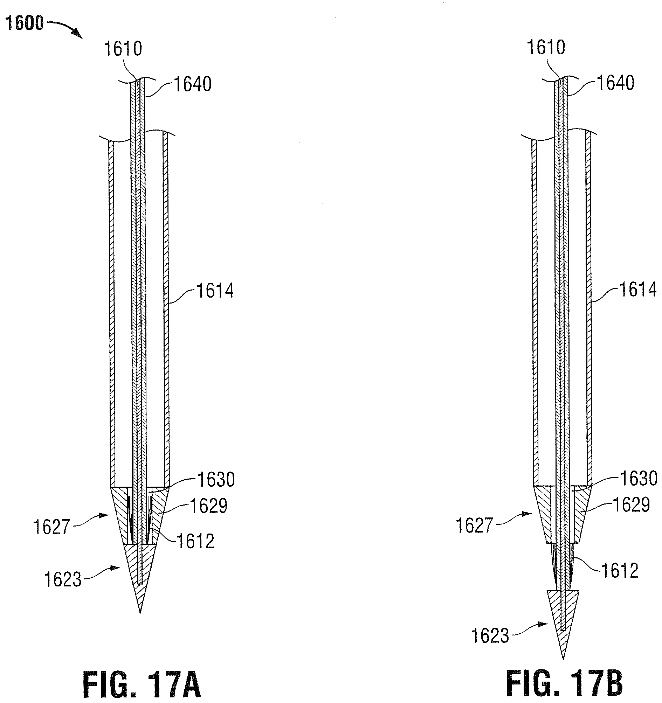

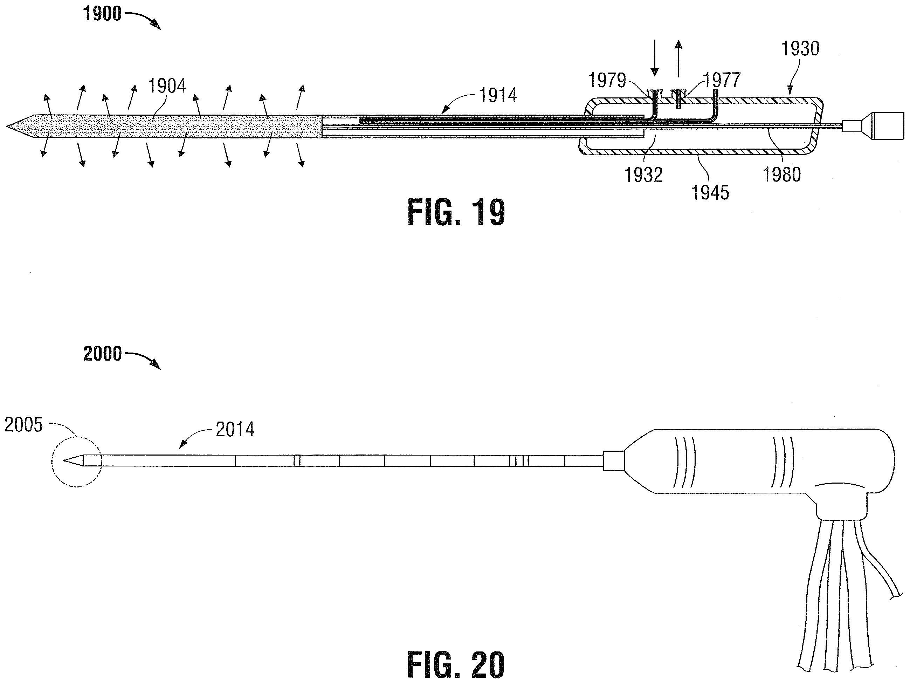

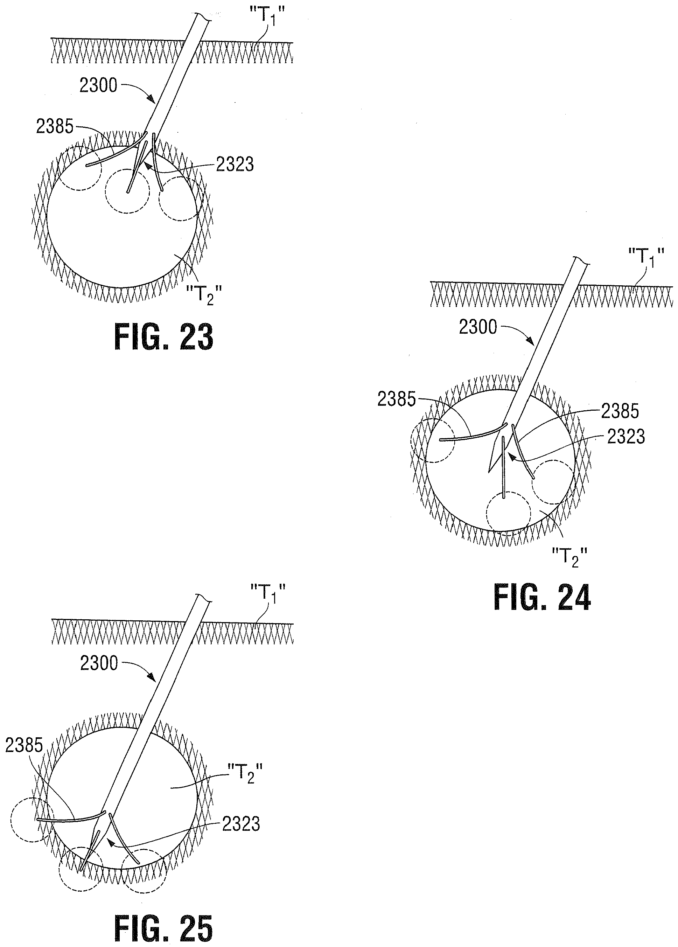

An embodiment of an ablation electrode assembly 101, similar to the ablation electrode assembly 110 of the electrosurgical system 100 shown in FIG. 1A, in accordance with the present disclosure, is shown in FIG. 1B. It is to be understood, however, that other ablation device (e.g., ablation system 200 shown in FIG. 2, ablation system 300 shown in FIG. 3, ablation device 400 shown in FIG. 4, ablation device 510 shown in FIGS. 5A and 5B, ablation needle 600 shown in FIGS. 6 and 7, ablation needle assembly 800 shown in FIG. 8, ablation needle 900 shown in FIG. 9, ablation needle assembly 1000 shown in FIG. 10, ablation needle assembly 1100 shown in FIG. 11, ablation needle assembly 1200 shown in FIG. 12, ablation device 1300 shown in FIGS. 13-14B, ablation device 1500 shown in FIG. 15, ablation device 1400 shown in FIGS. 16A and 16B, RF ablation device 1600 shown in FIGS. 17A-18B, ablation device 1900 shown in FIG. 19, ablation device 2000 shown in FIGS. 20-22, ablation needle 2300 shown in FIGS. 23-25, and combinations thereof) may also be used.

In some embodiments, electrosurgical system 100 (also referred to herein as ablation system 100) may include a controller 26 for controlling and/or monitoring the operating parameters of the ablation system 100. In some embodiments, as shown in FIG. 1A, the controller 26 is communicatively-coupled to the electrosurgical power generating source 28. Controller 26 may additionally, or alternatively, be communicatively-coupled to the fluid source 48. In some embodiments, the controller 26 may receive user-inputs from one or more user-input devices, such as without limitation, a keyboard, a pointing device, e.g., a mouse, joystick or trackball, a touchscreen, and/or other device communicatively-coupled to the controller 26. In some embodiments, electrosurgical system 100 includes an imaging system (not shown) capable of generating image data, and the controller 26 may be communicatively-coupled to the imaging system. Controller 26 may include any type of computing device, computational circuit, or any type of processor or processing circuit capable of executing a series of instructions that are stored in a memory (not shown) associated with the controller 26. Functions of the controller 26 may be performed in hardware and/or software, as desired. Controller 26 may include logic, circuitry and/or code adapted to control the electrosurgical power generating source 28 and/or the coolant source 48 responsive to one or more electrical signals received from one or more user-input devices. Functions of the controller 26 may be integrated with those of the electrosurgical power generating source 28, may be integrated with other components of the electrosurgical system 100, and/or may be in the form of stand-alone units coupled among components of the electrosurgical system 100.

As seen in FIGS. 1A and 1B, ablation electrode assembly 110 includes an elongated ablation needle 112. Ablation needle 112 includes a substantially cylindrically-shaped body or shaft portion 114 defining a cavity or 116 therein. Ablation needle 112 includes a distal end portion 118 including a tapered portion, which may terminate in a sharp tip 118a to allow for insertion into tissue with minimal resistance. Ablation needle 112 includes a proximal end portion 120, which may be configured for connection to a hub 130, which is described in more detail later in this description. Ablation needle 112 is fabricated from an electrically-conductive material, e.g., stainless steel, titanium, etc. The shape and size of the ablation needle 112 may be varied from the configuration depicted in FIGS. 1A and 1B.

Ablation electrode assembly 110 includes an insulative coating 122 over at least a portion of the length of the ablation needle 112. In some embodiments, the insulative coating 122 is disposed over substantially the length of the ablation needle 112. In some embodiments, as shown in FIGS. 1A and 1B, the insulative coating 122 extends from the hub 130 to the distal end portion 118 of the ablation needle 112, such that the distal end portion 118 of the ablation needle 112 is exposed or non-insulated. Insulative coating 122 is used to prevent the flow of electrical current from the shaft portion 114 of the ablation needle 112 into surrounding tissue. Insulative coating 122 shields the intervening tissue from RF current, so that such tissue is not substantially heated along the length of shaft portion 114 except by the heating effect from the distal end portion 118 which is exposed.

In some embodiments, as shown in FIGS. 1A and 1B, an ablation electrode assembly 110 includes one or more heat sinks, in the form of a heat strap or heat pipe 124 extending through the chamber 116, or portion thereof, of the ablation needle 112. It is to be understood, however, that in ablation electrode embodiments (e.g., electrode 211 of the ablation system 200 shown in FIG. 2) coolant fluid (and/or drug agent) may circulate to a tip portion for cooling of the electrode without the use of heat sinks.

Referring to FIGS. 1A and 1B, heat strap 124 includes a distal end 124a operatively secured to the ablation needle 112, and a proximal end 124b extending into a chamber 132 formed in the hub 130. In some embodiments, the distal end 124a of the heat strap 124 is operatively connected or secured to the distal end portion 118 of the ablation needle 112. In some embodiments, the distal end 124a of the heat strap 124 is bonded to the distal end portion 118 of the ablation needle 112 with a thermally-conductive adhesive or the like. Although a single heat strap 124 is shown in FIGS. 1A and 1B, a plurality of heat straps 124 may be provided. It is to be understood, however, that in ablation electrode embodiments (e.g., electrode 211 of the ablation system 200 shown in FIG. 2) coolant fluid (and/or drug agent) may circulate to a tip portion for cooling of the ablation electrode without the use of heat sinks.

Heat strap 124 is fabricated from a highly heat-conductive anisotropic material, e.g., graphite fiber. Accordingly, in use, the heat strap 124 draws heat away from distal end portion 118 of the ablation needle 112 and dissipates the heat along a length thereof. In order to increase the efficiency and the rate of heat dissipation, a cooling fluid may be circulated over the proximal end 124b of the heat strap 124.

Hub 130 may have a variety of suitable shapes, e.g., cylindrical, rectangular, etc. Hub 130 generally includes a hub body 145 defining a chamber 132 therein. In some embodiments, as shown in FIG. 1A, hub body 145 defines an outlet fluid port 177 and an inlet fluid port 179 disposed in fluid communication with the chamber 132. Hub 130 may include an inlet conduit 134 for delivering coolant fluid "F" through the inlet fluid port 179 into the chamber 132, and may include an outlet conduit 136 for delivering coolant fluid "F" through the outlet fluid port 177 from the chamber 132. In some embodiments, coolant chamber 132 may include baffles, multiple lumens, flow restricting devices, or other structures that may redirect, concentrate, or disperse flow depending on their shape. Examples of coolant chamber embodiments are disclosed in commonly assigned U.S. Patent Application Serial No. 12/350,292 filed on January 8, 2009, entitled "CHOKED DIELECTRIC LOADED TIP DIPOLE MICROWAVE ANTENNA", commonly assigned U.S. Patent Application Serial No. 12/401,268 filed on March 10, 2009, entitled "COOLED DIELECTRICALLY BUFFERED MICROWAVE DIPOLE ANTENNA", and U.S. Pat. No. 7,311,703, entitled "DEVICES AND METHODS FOR COOLING MICROWAVE ANTENNAS".

In operation, coolant fluid "F" is communicated into the chamber 132 through the inlet conduit 134 and out of the chamber 132 through the outlet conduit 136. Coolant fluid "F" may be any suitable fluid that can be used for cooling the ablation needle 112, e.g., deionized water, or other suitable cooling medium. As coolant fluid "F" is circulated through the chamber 132 of the hub 130, heat or energy is withdrawn from the proximal end 124b of the heat strap 124 and carried away with fluid flow, e.g., to the fluid source 48 for re-cooling and the like.

In some embodiments, as shown in FIG. 1A, the hub 130 may include a proximal connector (e.g., a luer connector) including a tapered hole 140 or the like. Into female luer connector 140, a hub of a high-frequency or thermo-sensing electrode 142 may be inserted and sealed by its male luer connection. A probe 144 of the thermo-sensing electrode 142 may be connected to the ablation needle 112, which may sense the temperature of ablation needle 112 at that point, or alternatively, may sense the temperature of the distal end portion 118.

Connected to or within the hub of the high-frequency and/or thermo-sensing electrode 142 are connections, indicated by dashed lines in FIG. 1A, which connect to the electrosurgical power generating source 28 and/or a thermal-sensing circuit "TC". In some embodiments, the thermal-sensing circuit "TC" may be of a thermocouple type, and the temperature sensor may a bi-metal junction thermocouple. The temperature sensor may be sensor capable of generating a signal indicative of a temperature of a medium in contact therewith.

Coolant source 48 may include any suitable housing containing a reservoir of coolant fluid "F". Coolant source 48 stores coolant fluid "F", and may maintain coolant fluid "F" at a predetermined temperature. For example, the coolant source 48 may include a cooling unit (not shown) that cools the returning coolant fluid "F" from the ablation electrode assembly 110. Ablation system 100 may include a coolant supply system (not shown) adapted to provide the coolant fluid "F", e.g., from the coolant source 48, to the ablation electrode assembly 110. In some embodiments, one or more components of a coolant supply system may be integrated fully or partially into the electrosurgical power generating source 28.

During ablation, e.g., using the electrosurgical system 100, the ablation electrode assembly 110 is inserted into or placed into the body of a patient, e.g., percutaneously or intraoperatively. A plurality of ablation electrodes 110 may be placed in variously arranged configurations to substantially simultaneously ablate a target tissue region, making faster procedures possible. Ultrasound or computed tomography (CT) guidance may be used to accurately guide the ablation electrode assembly 110 into the area of tissue to be treated. Electrosurgical power generating source 28 may be the source of high-frequency voltage which produces the high-frequency current that emanates from the distal end portion 118 of ablation needle 112. Following treatment or ablation of the target tissue, ablation electrode assembly 110 may be withdrawn from the target site and introduced into another target site, into the same target site from a different angle or approach, or in substantially the same location.

Examples of electrosurgical generators that may be suitable for use as the electrosurgical power generating source 28 include generators sold by Covidien Surgical Solutions of Boulder, CO, e.g., FORCE EZ™ electrosurgical generator, FORCE FX™ electrosurgical generator, and FORCE TRIAD™ electrosurgical generator FORCE 1C™ generator, FORCE 2™ generator, SurgiStat™ II, or other generators which may perform different or enhanced functions.

In alternative embodiments not shown, the ablation electrode assembly 110 may include an inflatable balloon member which may be connected to walls of the electrode assembly 110 using any fastening technique, e.g., adhesive, sonic welding, or by any other suitable process. The inflatable balloon member (not shown) may be operated in conjunction with the delivery of a drug agent. The walls of the electrode assembly 110 may be provided with an opening or port disposed and configured to place a inflation lumen in fluid communication with the inflatable balloon member, e.g., to allow drug-delivery flow supplied via the inflation lumen to be used to operate the inflatable balloon member, e.g., drug-eluting balloon. In some embodiments, the electrode assembly 110 may be adapted to allow user control of operational characteristics of the drug-eluting balloon, e.g., rate of inflation, inflation volume, and pressure exerted by the inflatable balloon member on the tissue surrounding the inflatable balloon member.

FIG. 2 shows an ablation system 200 including an energy applicator 201 according to an embodiment of the present disclosure. Energy applicator 201 includes an elongated shaft or cannula body "C" for positioning in tissue, e.g., percutaneously or intraoperatively into an open wound site. In some embodiments, the cannula body "C" is integral with a hub "H" coupled to remote support components, collectively designated "S". Energy applicator 201 is operatively connected to an electrosurgical power generating source 28, e.g., a microwave or radio frequency (RF) electrosurgical generator.

Cannula body "C" includes an elongated ablation electrode 211 formed of conductive material, e.g. metal such as stainless steel, titanium, etc. Electrode 211 may include a substantially hollow tubular body sized in length and diameter to fit within the cannula body "C". At the distal end of the cannula body "C", the electrode 211 defines a tip 212. In operation when using an RF power supply 216, electrical current spreads from the tip 212 to pass through the surrounding tissue causing the tissue to heat up.

Electrode 211 carries an insulative coating 213 over a portion of its length for selectively preventing the flow of electrical current from the shaft 215 of electrode 211 into surrounding tissue. Insulative coating 213 shields the intervening tissue from RF current, so that tissue along the length of the shaft 215 is not substantially heated except by the heating effect from the exposed tip 212.

The proximal end of the electrode 211 is integral with an enlarged housing 214 of the hub "H", which carries electrical and coolant connections as described below. In the portion disposed outside the patient's body, the housing 214 is of cylindrical configuration, defining ports for connections to the support components "S", e.g., electrical and fluid couplings. Housing 214 may be integral with the electrode 211, formed of metal, or it may constitute a separate subassembly as described below. Housing 214 may be formed of plastic, and may accommodate separate electrical connections. In that regard, a plastic housing 214 is amenable to low artifact imaging by X-rays CT, MRI, etc. as may be desirable in some situations.

The housing 214 mates with a block 218 defining a luer taper lock 219 sealing the block 218 to the housing 214. Connection to a regulated RF power source 216 may take the form of a standard cable connector, a leader wire, a jack-type contact or other designs. The temperature-sensing and radiofrequency electrical connections may be made through the housing 214 and extend to the region of the tip 212, where an RF line 225 is connected by junction 221, e.g., a weld, braze, or other secure electrical connection. In some embodiments, sensor lines 224 extend to a thermo-sensor 223, e.g., a thermistor, or a thermocouple, or any other type of temperature sensing device capable of sending a signal indicative of a temperature. Thermo-sensor 223 may be fused or in thermal contact with the wall of the tip 212 to sense the temperature of the tip 212.

RF power source 216 may be referenced to reference potential, as illustrated FIG. 2, and coupled through the block 218 affixed to the hub "H". In embodiments, the RF power source 216 provides RF voltage through the block 218 with an electrical connection to the electrode 211 as indicated by the line 225, to the connection junction 221. RF power source 216 may take the form of an RF generator. Examples of RF generators that may suitably be used as the RF power source 216 may include the RFG-3C RF Lesion Generator System available from Radionics, Inc., Burlington, Massachusetts.

As indicated above and in accordance with common practice, when the ablation electrode 211 is in a patient's body, an electrical circuit is completed through the body to a reference or dispersive electrode R (symbolically represented in FIG. 2) that is connected elsewhere to the body. Energy transmitted from the RF power source 216 heats body tissue by current from the tip 212. In that regard, a temperature monitor 220 may be electrically connected by lines 222 and 224 to a temperature sensor 223 disposed within or contacting the tip 212. Temperature sensor 223 may be a thermocouple, thermistor, or other temperature sensing device. In an embodiment, the sensor 223 is connected to the tip 212. The sensed temperature may be utilized to control either or both of the flow of RF energy or the flow of coolant to attain the desired ablation while maintaining the maximum temperature substantially below a predetermined temperature, e.g., 100° C. One or more sensors may be utilized to measure temperatures at various locations in the proximity of the tip 212. One or more sensor devices, or components thereof, may be disposed outside the distal end portion 118 of the ablation needle 212. Examples of temperature monitoring devices that may suitably be used as the temperature monitor 220 may include the TC thermocouple temperature monitoring devices available from Radionics, Inc., Burlington, Massachusetts.

In accordance herewith, temperatures at, or near the tip 212 (e.g., manifest by the temperature monitor 220) may be controlled by controlling the flow of coolant fluid through the ablation electrode 211. In this manner, the temperature of the surface area of the tip 212 in contact with tissue is controllable. In an embodiment, fluid from a fluid source "FS" is carried the length of the ablation electrode 211 through a tube 226 extending from the housing 214 to the distal end of the electrode 211 terminating in an open end 228 at the tip 212. At the proximal end of the electrode 211, within the housing 214, the tube 226 is connected to receive coolant fluid. Fluid flow may be regulated in accordance with the sensed temperature sensed at the tip 212, allowing increased flow of RF energy.

The fluid coolant may take the form of water or saline for the convection removal of heat from the tip 212. A reservoir or source unit for supplying coolant fluid may be a large reservoir of cooled water, saline or other fluid. As an illustrative example, a tank of water with ice cubes can function to maintain the coolant at a temperature of approximately 0° C. As another example, the fluid source "FS" could incorporate a peristaltic pump or other fluid pump, or could merely be a gravity feed for supplying fluid from a bag or rigid tank.

Flow from the tip 212 to the hub "H" exits the hub "H" through an exit port 240 as illustrated by arrows 242 and 243. The port 240 may take the form of couplings, rigid units or may include flexible tubular couplings to reduce torque transmission to the electrode 211. The coolant flow members may take the form of PVC tubes with plastic luer connectors for ease of use.

As a result of the coolant flow, the interior of the electrode 211, e.g., the electrode tip 212, can be maintained at a temperature near that of the fluid source "FS". The coolant may circulate in a closed system as illustrated in FIG. 2. In some situations, it may be desirable to reverse the direction of fluid flow from that depicted in the FIG. 2. Coordinated operation involving RF heating along with the cooling may be accomplished by a controller, e.g., microprocessor 244. In some embodiments, the microprocessor 244 may be coupled to the RF power source 216, the temperature monitor 220 and the fluid source "FS" to receive data on flow rates and temperatures and adapted to control operating parameters of support components "S". An integrated operation may be provided with feedback from the temperature monitor 20 in a controlled format and various functions can be concurrently accomplished. Such controlled operation may effectively reduce the temperature of tissue near the tip 212 to accomplish an equilibrium temperature distribution tailored to the size of the targeted tumor.

The temperature distribution in the tissue near the tip 212 generally depends on the RF current from the radiating section "R" and/or tip 212 and on the temperature of the tissue which is adjacent to the radiating section "R" and/or tip 212. The tip temperature can be controlled to approach the temperature of the fluid from the source "FS". In this manner, a thermal boundary condition may be established, holding the temperature of the tissue near the radiating section "R" and/or tip 212 to approximately the temperature of the tip itself, e.g., the temperature of the coolant fluid inside the tip 212. Accordingly, by temperature control, a surgeon may impose a defined temperature at the boundary of the electrode radiating section "R" and/or tip 212 which can be somewhat independent of the RF heating process and may significantly modify the temperature distribution in the tissue.

FIG. 3 shows an ablation system 300 in accordance with an embodiment of the present disclosure that includes an electrode array "E". Electrode array "E" may include one or more ablation electrodes 110. Electrode array "E" is operatively connected to the electrosurgical power generating source 28, and may be disposed in fluid communication with the coolant source 48. Power generating source 28 may be any generator suitable for use with electrosurgical devices and may be configured to provide various frequencies of energy.

In some embodiments, as shown in FIG. 3, the electrode array "E" includes three ablation electrodes 110 supported on and/or operatively connected to a hub element 330. Hub 330 may be similar to the hub "H" shown in FIG. 2 (or hub 130 shown in FIGS. 1A and 1B) and further description thereof is omitted in the interests of brevity. Insulative coating 122 extends from the hub 330 to the distal end portion 118 of the ablation needle 112 of each ablation electrode assembly 110. The shape, size and number of ablation electrodes 110 of the electrode array "E" may be varied from the configuration depicted in FIG. 3.

FIG. 4 shows an ablation system 400 in accordance with another embodiment of the present disclosure that includes an electrode array "E". Electrode array "E" may include one or more ablation electrodes 110 adapted to allow drug delivery through the ablation needles 112 thereof to tissue. Electrode array "E" is operatively connected to an electrosurgical power generating source 28, and may be disposed in fluid communication with a drug reservoir 448. In some embodiments, as shown in FIG. 4, the distal end portion 418 of the ablation needles 112 of the ablation electrodes 110 include one or more drug delivery ports 430. In other embodiments, the radiating section of an ablation device (e.g., ablation device 1900 shown in FIG. 19) may include an antenna assembly including a porous-metal radiating section (e.g., radiating section 1904 shown in FIG. 19) suitable for drug delivery to tissue.

FIGS. 5A and 5B show an electrosurgical system (shown generally as 500) in accordance with an embodiment of the present disclosure that includes an ablation device 510 including a plurality of the ablation electrodes 110 and a delivery needle 401. Ablation device 510 includes a handle assembly 450. Ablation device 510 is adapted to allow the user to selectively position the delivery needle 401 in tissue. For ease of explanation and understanding, the delivery needle 401 is described below as selectively positionable with respect to fixed structures, or portions thereof, of the ablation device 510, e.g., in relation to the distal end portion 118 of the ablation needles 112 and/or in relation to the distal end 417 (FIG. 5B) of the handle assembly 450.

In some embodiments, as shown in FIGS. 5A and 5B, ablation device 510 is adapted to allow the user to selectively position at least the distal end 423 of the delivery needle 401 from at least a first configuration, wherein the distal end 423 of the delivery needle 401 is positioned proximal to the distal end portion 118 of the ablation needles 112, to at least a second configuration, wherein at least the distal end 423 of the delivery needle 401 is positioned distally beyond the distal end portion 118 of the ablation needles 112.

Handle assembly 450 generally includes a handle body 451 configured to support the ablation electrodes 110 and the delivery needle 401 at the distal end 417 thereof. Handle assembly 450 includes a slideably moveable member 460 adapted to allow the user to selectively move the delivery needle 401, e.g., from at least the first configuration to at least the second configuration. Slideably moveable member 460 may include a button 461 having a desired ergonomic form operably associated with the handle body 451. The button 461 may be configured to allow the user to selectively initiate/activate the delivery of drug and/contrast agent from the supply line 414 to the integral needle 401.

Handle assembly 450 may have various configurations. In some embodiments, the handle body 451 defines therein a handle-body chamber 476 having an interior space configured to accommodate one or more components of the ablation device 510, e.g., a hub (e.g., hub "H" shown in FIG. 2, or hub 330 shown in FIG. 3). Handle body 451 may include one or more internal walls (not shown) configured to partition the handle-body chamber 476 into one or more compartments. Handle assembly 450 may be formed of any suitable material or combination of materials by any suitable process. In some embodiments, the ablation device 510 may be adapted to be a reusable device. Autoclavable materials may be used to form the housing 451, and/or other components of the ablation device 510, to provide for a sterilizable device.

Handle assembly 450 or portions thereof, may be formed from two housing halves (not shown). Each half of the housing may include a series of mechanical interfacing components (not shown) configured to matingly engage with a corresponding series of mechanical interfaces (not shown) to align the two housing halves about the inner components and assemblies of the ablation device 510. It is contemplated that the housing halves (as well as other components described herein) may be assembled together with the aid of alignment pins, snap-like interfaces, tongue and groove interfaces, locking tabs, adhesive ports, etc., utilized either alone or in combination for assembly purposes.

Ablation electrodes 110 are operatively connected to the electrosurgical power generating source 28, and may be disposed in fluid communication with the coolant source 48. A transmission line 15 may be provided to electrically-couple the ablation device 510 to an electrosurgical power generating source (e.g., electrosurgical power generating source 28). Transmission line 15 may additionally provide a conduit (not shown) configured to provide coolant from a coolant source, e.g., deionized water, or other suitable cooling medium, for cooling one or more components of the ablation device 510, such as the ablation electrodes 110. In some embodiments, as shown in FIGS. 5A and 5B, a coolant supply line 18 leads from the handle assembly 450 to a coolant source (e.g., coolant source 48 shown in FIG. 1A).

A drug and/or contrast agent supply line 414 may be provided to fluidly-couple the ablation device 510 to a source of the drug and/or contrast agent delivery supply for supplying drugs and/or contrast agent to the handle assembly 450 and/or the integral needle 401. Handle assembly 450 may include one or more fluid conduits (not shown) associated with the handle body 451 configured to provide fluid communication between the supply line 414 and the integral needle 401. Transmission line 15 may additionally, or alternatively, provide a conduit (not shown) configured to provide drugs and/or contrast agent from a source of the supply line 414 to the handle assembly 450 and/or the integral needle 401.

In some embodiments, handle-body chamber 476 may include an interior space configured to accommodate a housing (not shown) containing a reservoir of drugs. In such case, handle body 451 may be provided with an opening covered by a removable cover plate, e.g., to allow removal of the housing containing a reservoir of the drug delivery supply.

In some embodiments, electrosurgical system 500 (also referred to herein as ablation system 500) may include a controller 26 for controlling and/or monitoring the operating parameters of the ablation system 500. In some embodiments, as shown in FIGS. 5A and 5B, the controller 26 is communicatively-coupled to the electrosurgical power generating source 28. In alternative embodiments not shown, ablation device 510 may include a user interface, e.g., configured to provide user-input capabilities and/or capabilities for simplified use and/or programming of the ablation device 510 and/or the electrosurgical power generating source 28. Some examples of operating parameters associated with the power generating source 28 that may be adjusted include temperature, impedance, power, current, voltage, mode of operation, and duration of application of electromagnetic energy. The user interface may be adapted to enable a user to selectively configure one or more operating parameters of the ablation device 510, or component thereof, e.g., depending upon a particular purpose and/or to achieve a desired surgical outcome.

FIGS. 6A and 6B show a portion of an ablation needle (shown generally as 600) in accordance with an embodiment of the present disclosure that includes a substantially cylindrically-shaped body or shaft portion 614 with an integral needle passageway 611 and a delivery needle 601, e.g., for the delivery of active pharmaceutical ingredients (APIs) and/or contrast agent, etc. Ablation needle 600 is configured to allow the user to selectively position the delivery needle 601, or portion thereof, from within the body or shaft portion 614 of the ablation needle 600 to outside the body or shaft portion 614.

Shaft portion 614 defines therein a first fluid-flow path 636, a second fluid-flow path 634 fluidly-coupled to the first fluid-flow path 636, and the needle passageway 611 of generally tubular shape configured to receive the delivery needle 601 slideably moveably therein. Although the passageway 611 is generally tubular-shaped, other shapes can be used depending on the configuration of the delivery needle 601.

As seen in FIGS. 6A and 6B, delivery needle 601 is selectively moveable from at least a first configuration, wherein the distal end 623 of the delivery needle 601 is positioned proximal to the distal end portion 618 of the ablation needle 600 (FIG. 6A), to at least a second configuration, wherein at least the distal end 623 of the delivery needle 601 is positioned distally beyond the distal end portion 618 of the ablation needle 600 (FIG. 6B). In some embodiments, the delivery needle 601 may be advanced and retracted by way of a thumb-slide actuator, or the like, which may be adapted to keep the delivery needle retracted during the ablation portion of the procedure, then extended post ablation to administer the drug and/or contrast agents to the target site.

First fluid-flow path 636, e.g., leading to the distal end portion 618 of the ablation needle 600, and the second fluid-flow path 634, e.g., leading away from the distal end portion 118, are configured to provide fluid flow of a coolant fluid "F", e.g., deionized water, or other suitable cooling medium, for cooling at least the distal end portion 618 of the ablation needle 600. In some embodiments, first fluid-flow path 636 and/or the second fluid-flow path 634 are fluidly-coupled to a hub (e.g., hub 130 shown in FIGS. 1A and 1B, hub "H" shown in FIG. 2, or hub 330 shown in FIG. 3) providing at least one coolant connection to the ablation device 600 for providing fluid flow of the coolant fluid "F" for cooling the body or shaft portion 614 and/or other components of the ablation device 600.

Ablation needle 600 may be configured to be operatively coupleable to a handle assembly of an ablation device, which may be configured to support the ablation needle 600. In some embodiments, the ablation device (e.g., ablation device 510 shown in FIGS. 5A and 5B) may include one or more components, such as without limitation, the handle body 451 and the slideably moveable member 460 to allow the user to selectively move the delivery needle 612.

The delivery needle 601 may include micro-needle arrays disposed in association with a surface of the delivery needle 601. Micro-needle arrays may be made from electrical and/or temperature sensitive materials, and may be oriented in any suitable manner.

FIGS. 7A and 7B show a portion of an ablation needle (shown generally as 700) in accordance with an embodiment of the present disclosure that includes a substantially cylindrically-shaped body or shaft portion 614 with an integral passageway 611 and an antenna assembly 2014 adapted to be slideably moveable within the passageway 611. Antenna assembly 2014 includes a delivery needle 2023 adapted to be slideably moveable within the antenna assembly, e.g., for the delivery of active pharmaceutical ingredients (APIs) and/or contrast agent, etc.

As seen in FIGS. 7A and 7B, antenna assembly 2014 is selectively moveable from at least a first configuration, wherein the distal end of the antenna assembly 2014 is positioned proximal to the distal end portion 618 of the ablation needle 600 (FIG. 7A), to at least a second configuration, wherein at least the distal end of the antenna assembly 2014 is positioned distally beyond the distal end portion 618 of the ablation needle 600 (FIG. 7B). In some embodiments, the antenna assembly 2014 and/or the delivery needle 2023 may be advanced and retracted by way of a thumb-slide actuator, or the like, which may be adapted to keep the antenna assembly 2014 and/or the delivery needle 2023 retracted during the ablation portion of the procedure, then extended post ablation to administer the drug and/or contrast agents to the target site.

FIG. 8 shows a portion of an ablation needle assembly (shown generally as 800) in accordance with an embodiment of the present disclosure that includes an interchangeable sleeve member 870 including a plurality of drug reservoir divots 831 associated therewith. Ablation needle assembly 800 includes a substantially cylindrically-shaped body or shaft portion 814. Sleeve member 870 includes a generally tubular-shaped sleeve body 817 defining a plurality of recesses 830 therein. The recesses 830 may be formed in any suitable shape, and may define receptacles of any suitable volume to contain one or more drugs. The recesses 830 may have any suitable depth less than a through-penetration depth. Sleeve member 870 may be configured to be disposed coaxially around the body or shaft portion 814, or portion thereof. Sleeve member 870 may be either disposable or reusable.

The recesses 830 are provided with one or more drugs, which may be temperature-sensitive, therein. In some embodiments, the recesses 830 are provided with microspheres, e.g., API or CTA microspheres and/or microparticles, and may be provided with a thermo-sensitive binding agent, e.g., wax. In some embodiments, the recesses 830 are provided with one or more chemotherapeutic agents, and may be provided with a thermo-sensitive binding agent. A thermo-sensitive binding agent may be combined with the microspheres, API, or CTA disposed in the recesses 830. A thermo-sensitive binding agent may additionally, or alternatively, be formed as layered coating to protect and/or postpone delivery of the microspheres, API, or CTA.

In some embodiments, as shown in FIG. 8, a first drug 834 is disposed within one or more of the recesses 830, and a second drug 836 may be disposed within one or more of the recesses 830. Any suitable number of the same or different drug reservoir divots 831 may be utilized, e.g., depending upon a particular purpose and/or to achieve a desired surgical outcome. In some embodiments, one or more drug reservoir divots 831, e.g., containing the first drug 834, may be configured to be released at a first temperature (or first temperature range), and one or more drug reservoir divots 831, e.g., containing the second drug 836, may be configured to be released at a second temperature (or second temperature range). The shape, size, position and number of the recesses 830 may be varied from the configuration depicted in FIG. 8.

FIG. 9 shows a portion of an ablation needle (shown generally as 900) in accordance with an embodiment of the present disclosure that includes a plurality of drug reservoir divots 931 associated therewith. Any suitable number of the same or different drug reservoir divots 931 may be utilized. Ablation needle 900 includes a substantially cylindrically-shaped body or shaft portion 914 defining a plurality of recesses 930 therein.

The recesses 930 are provided with one or more drugs 834 therein, such as without limitation, microspheres, chemotherapeutic agents, and/or a thermo-sensitive binding agent, e.g., wax. Recesses 930 are similar to the recesses 830 shown in FIG. 8 and further description thereof is omitted in the interests of brevity. The shape, size, position and number of the recesses 930 may be varied from the configuration depicted in FIG. 9.

FIG. 10 shows a portion of an ablation needle assembly (shown generally as 1000) in accordance with an embodiment of the present disclosure that includes an interchangeable sleeve member 1070 including a plurality of drug reservoir divots 1031 associated therewith. Ablation needle assembly 1000 includes a substantially cylindrically-shaped body or shaft portion 1001 formed of an electrically-conductive material, e.g., stainless steel, titanium, etc. Body or shaft portion 1001 is operatively connected to an electrosurgical power generating source (e.g., electrosurgical power generating source 28 shown in FIG. 1A).

Sleeve member 1070 is configured to be disposed coaxially around at least a portion of the body or shaft portion 1001. Recesses 1030 may be provided with one or more drugs 834 therein. Any suitable number of the same or different drug reservoir divots 1031 may be utilized.

FIG. 11 shows a portion of an ablation needle assembly (shown generally as 1100) in accordance with an embodiment of the present disclosure that includes an interchangeable sleeve member 1170 including a plurality of drug reservoir divots 1131 associated therewith. Ablation needle assembly 1100 includes an elongated substantially cylindrically-shaped body or shaft portion 1001 formed of an electrically-conductive material, e.g., stainless steel.

The body or shaft portion 1001 is operatively connected to an electrosurgical power generating source (e.g., electrosurgical power generating source 28 shown in FIG. 1A). The power generating source may include any generator suitable for use with electrosurgical devices, and may be configured to provide various frequencies of electromagnetic energy. In some embodiments, the electrosurgical power generating source is configured to provide microwave energy at an operational frequency from about 300 MHz to about 10 GHz.

Sleeve member 1170 may be configured to be disposed coaxially around the body or shaft portion 1001, or portion thereof. Recesses 1130 may be provided with one or more drugs 834 therein. Any suitable number of the same or different drugs may be utilized, e.g., depending upon a particular purpose and/or to achieve a desired surgical outcome.

Sleeve member 1170 additionally, or alternatively, includes one or more apertures 1190 defined therethrough. Apertures 1190 are configured to allow electromagnetic energy, e.g., microwave energy, to be delivered to tissue. In some embodiments, as shown in FIG. 11, the apertures 1190 are axially aligned along the longitudinal axis of the body or shaft portion 1001, e.g., to provide a directional radiation pattern. The shape, size, position and number of the recesses 1130 and the apertures 1190 may be varied from the configuration depicted in FIG. 11.

FIG. 12 shows a portion of an ablation needle assembly (shown generally as 1112) in accordance with an embodiment of the present disclosure that includes an interchangeable sleeve member 1270 including a plurality of drug reservoir divots 1231 associated therewith. Ablation needle assembly 1112 includes a substantially cylindrically-shaped body or shaft portion 1001 formed of an electrically-conductive material. Body or shaft portion 1001 is electrically-coupled to an electrosurgical power generating source (e.g., electrosurgical power generating source 28 shown in FIG. 1A).

Sleeve member 1270 may be configured to be disposed coaxially around the body or shaft portion 1001, or portion thereof. Recesses 1230 may be provided with one or more drugs 834 therein. Any suitable number of the same or different drugs may be utilized.

As seen in FIG. 12, sleeve member 1270 includes one or more elongated apertures or slots 1290 defined therethrough. Slots 1290 are configured to allow electromagnetic energy, e.g., microwave energy, to be delivered to tissue. The shape, size, position and number of the recesses 1230 and the slots 1290 may be varied from the configuration depicted in FIG. 12.

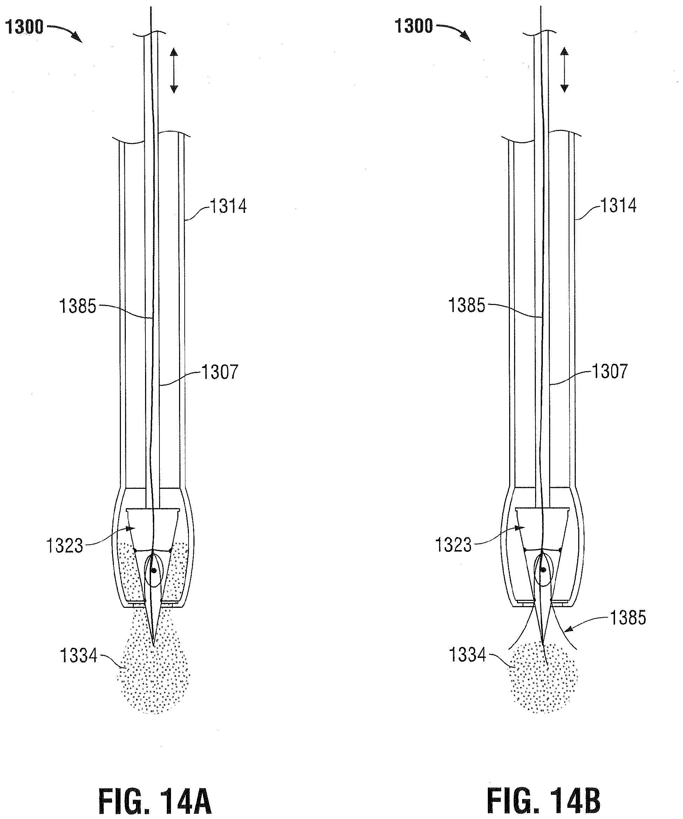

FIGS. 13, 14A and 14B show an RF ablation device (shown generally as 1300) in accordance with an embodiment of the present disclosure that includes a drug reservoir 1348 and a moveable tip portion 1323 containing one or more deployable tendrils and/or projections 1385. Ablation device 1300 includes an elongated body or shaft portion 1340 and a rod member 1307 disposed therein. Rod member 1307 defines a lumen configured to allow one or more deployable tendrils and/or projections 1385 to pass therethrough. Tip portion 1323 includes one or more apertures 1390 defined therethrough, e.g., configured to allow the tendrils to pass therethrough. The lumen defined by the rod member 1307 may be disposed in communication with the one or more apertures 1390 defined by the tip portion 1323.

Rod member 1307 is coupled at its distal end to the tip portion 1323 and configured to be slideably moveable within the shaft portion 1340. This arrangement permits relative longitudinal movement of the rod member 1307 to effect movement of the tip portion 1323. As seen in FIGS. 14A and 14B reverse axial movement of the rod member 1307 into the body or shaft portion 1340 moves at least a portion of the tip portion 1323 into the drug reservoir 1348.

Although five tendrils and/or projections 1385 are shown in FIG. 13, the number of tendrils and/or projections 1385 may vary, e.g., depending upon a particular purpose and/or to achieve a desired surgical outcome. In some embodiments, the tendrils and/or projections 1385 may be configured to inject API, CTA, and/or suspended microspheres. The tendrils and/or projections 1385 may additionally, or alternatively, have a coating thereon to facilitate penetration into the tissue and/or tumor.

In some embodiments, the tendrils and/or projections 1385 may additionally, or alternatively, be configured to break off and become implanted into target tissue, e.g., tumor. The tendrils and/or projections 1385 may be implanted permanently, or until degraded, dissolved and/or absorbed. In some embodiments, the tendrils and/or projections may be made of biodegradable material to degrade over time while releasing one or drugs. In some embodiments, the tendrils and/or projections 1385 may have a micro-needle array on the surface of the tendrils and/or projections to further penetrate the tissue and/or tumor with drug (e.g., API, CTA, and/or suspended microspheres).

As seen in FIGS. 13, 14A and 14B, the tip portion 1323 is selectively moveable from a first configuration (FIG. 13) to at least a second configuration, wherein at least a portion of the tip portion 1323 is disposed within the drug reservoir 1348 during the delivery of drugs from the drug reservoir 1348 to tissue (FIG. 14). In some embodiments, the tendrils 1385 may be controlled by a center braided cord 1385, either electrically or mechanically. One or more tendrils 1385 may be deployed before the drug is released from the reservoir 1348. One or more tendrils 1385 may additionally, or alternatively, be deployed as the drug is released from the reservoir 1348. One or more tendrils 1385 may additionally, or alternatively, be deployed after the drug has been released. For example, after the drug is released as shown in FIG. 14, the device can move back to the first configuration (FIG. 13) to deploy the tendrils 1385.

FIG. 15 shows a distal portion of an ablation device 1500 in accordance with an embodiment of the present disclosure that includes a tip portion 1523 including a plurality of openings or ports 1590 defined therethrough and a configuration of deployable tendrils and/or projections 1585 disposed within the tip portion 1523. The tendrils and/or projections 1585 are configured to be deployable through the ports 1590 into tissue. In some embodiments, the ports 1590 are configured to allow the tendrils and/or projections 1585 to be deployable at about 90° relative to the surface of the tip portion 1523. The configuration shown in FIG. 15 provides all access to the tumor/lesion, because the tendrils are deployed at different heights of the tip portion 1523 and the tendrils exit perpendicular to each other at 90 degree angles around the circumference of the tip portion 1523. In some embodiments, four tendrils are connected to a center braided cord (e.g., 1385 shown in FIGS. 13-14B) that activates the deployment, either electrically or mechanically.

FIG. 16A shows a distal portion of an ablation device 1400 in accordance with an embodiment of the present disclosure that includes a body or shaft portion 1414 defining a cavity or chamber 1416 therein and including an open end 1415 in communication with the chamber 1416. A moveable member 1407, e.g., cylindrically-shaped, is disposed within the body or shaft portion 1414 and adapted to be longitudinally-moveable therein. A plurality of tendrils 1485 is disposed in association with the moveable member 1407. In some embodiments, as shown in FIG. 16A, at least a portion of the tendrils 1485 extend outwardly of the distal end 1408 of the moveable member 1407.

Tendrils 1485 may be made of biodegradable material to degrade over time while releasing one or drugs. In some embodiments, the tendrils 1485 may be configured to inject API, CTA, and/or suspended microspheres. Tendrils 1485 may additionally, or alternatively, have a coating thereon to facilitate penetration into the tissue and/or tumor. In some embodiments, the tendrils 1485 may include one or more micro-needle arrays associated therewith to further penetrate the tissue and/or tumor with drug (e.g., API, CTA, and/or suspended microspheres). Tendrils 1485 may additionally, or alternatively, be configured to break off and become implanted into target tissue, e.g., tumor.

Moveable member 1407 is selectively moveable from at least a first configuration, as seen in FIG. 16A, wherein the distal end 1408 of moveable member 1407 is positioned proximal to the distal end portion 1418 of the body or shaft portion 1414, wherein the tendrils 1485 are contained within the shaft portion 1414, to at least a second configuration, as seen in FIG. 16B, wherein the distal end 1408 of moveable member 1407 is positioned adjacent or near the distal end portion 1418 of the body or shaft portion 1414, wherein at least a portion of the tendrils 1485 extend outwardly of the open end 1415 of the body or shaft portion 1414 into tissue.

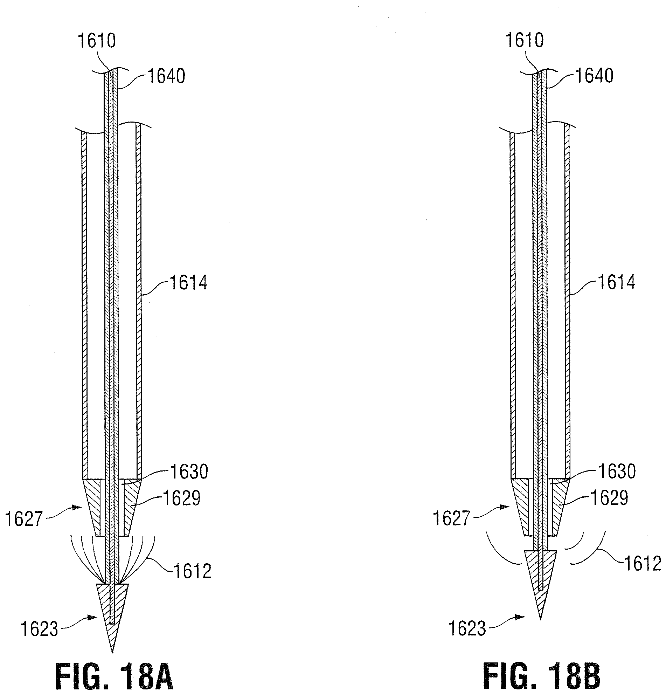

FIGS. 17A, 17B, 18A and 18B show of a portion of an RF ablation device (shown generally as 1600 in FIG. 17A) in accordance with an embodiment of the present disclosure that includes deployable RF antenna filaments 1612. RF ablation device 1600 includes an inner conductor 1610 formed of any suitable electrically-conductive material, a dielectric material 1640 coaxially disposed around the inner conductor 1610, and a moveable tip portion 1623 coupled to the distal end of the inner conductor 1610. RF ablation device 1600 includes a barrel portion 1627 configured to house the RF antenna filaments 1612, which are selectively deployable therefrom. One or more antenna filaments 1612 may be adapted to release one or more drugs into tissue. In some embodiments, one or more antenna filaments 1612 may be coated with one or more drugs. In some embodiments, one or more antenna filaments 1612 may include a plurality of drug reservoir divots (e.g., drug reservoir divots 931 shown in FIG. 9) associated therewith.

Barrel portion 1627 defines a chamber 1630 therein. The chamber 1630 is configured to house the RF antenna filaments 1612. The antenna filaments 1612 can break off to act as implantable filaments. The mechanism of the antenna filaments breaking off may be done in two steps: (1) the inner conductor 1610 is moved distally from the moveable tip portion 1623; and (2) the antenna filaments 1612 break off because of the tension that is created between the top edge of the moveable tip 1623 and the inner conductor 1610. The top edge of the moveable tip 1623 may additionally, or alternatively, be machined to have a razor sharp edge to help facilitate the separation of the antenna filaments 1612.

Referring to FIG. 17A, the tip portion 1623 is disposed in a second configuration, during deployment of the RF antenna filaments 1612 into tissue. In the second configuration shown in FIG. 17B, the tip portion 1623 is spaced apart from the barrel portion 1627 to allow the RF antenna filaments 1612, which are attached in part to the tip portion 1623, to deploy from the chamber 1630.

Referring to FIG. 18A, the tip portion 1623 is disposed in a third configuration wherein the antenna filaments 1612 are deployed with the proximal ends thereof expanded outward and the distal ends thereof attached to the tip portion 1623.

Referring to FIG. 18B, the tip portion 1623 is disposed in a fourth configuration wherein one or more of the antenna filaments 1612 are separated from the tip portion 1623, e.g., embedded in the tumor.

FIG. 19 shows an ablation device (shown generally as 1900) in accordance with an embodiment of the present disclosure that includes an antenna assembly 1914 including a porous-metal radiating section 1904 suitable for delivery of drugs (as indicated by arrowed lines) to tissue. Radiating section 1904 is disposed in fluid communication with a drug reservoir (e.g., drug reservoir 448 shown in FIG. 4) via a drug delivery conduit 1980. Antenna assembly 1914 is operatively connected to an electrosurgical power generating source (not shown) e.g., a microwave or RF electrosurgical generator, and may be disposed in fluid communication with a coolant source (e.g., coolant source 48 shown in FIG. 1A).

In some embodiments, ablation device 1900 may include a hub 1930. Hub 1930 may provide electrical and/or coolant connections to the antenna assembly 1914, and may be configured to support the antenna assembly 1914. Hub 1930 may have a variety of suitable shapes, e.g., cylindrical, rectangular, etc. Hub 1930 generally includes a hub body 1945 defining a chamber 1932 therein. In some embodiments, as shown in FIG. 19, hub body 1945 defines an outlet fluid port 1977 and an inlet fluid port 1979 disposed in fluid communication with the chamber 1932.

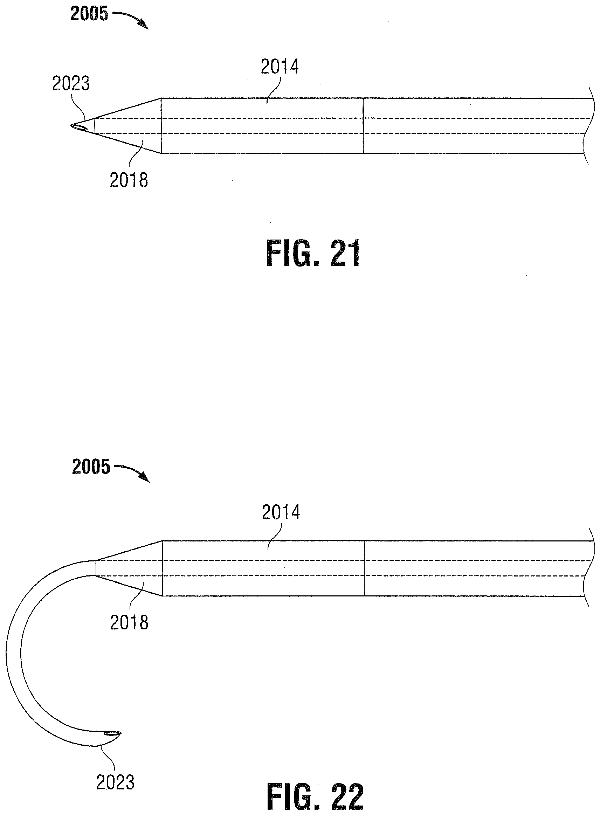

FIG. 20 shows an ablation device (shown generally as 2000) in accordance with an embodiment of the present disclosure that includes an antenna assembly 2014 having a distal end portion 2005. As shown in FIGS. 21 and 22, the antenna assembly 2014 includes a delivery needle 2023 adapted to be slideably moveable within the antenna assembly 2014, e.g., for the delivery of active pharmaceutical ingredients (APIs) and/or contrast agent, etc.

FIG. 21 shows the distal end portion 2005 of the antenna assembly 2014 including the delivery needle 2023 shown in a first configuration in accordance with an embodiment of the present disclosure. Antenna assembly 2014 includes a distal end 2018 including a tapered portion, which may terminate in a sharp tip to allow for insertion into tissue with minimal resistance. The shape and size of the antenna assembly 2014 may be varied from the configuration depicted in FIGS. 1A and 1B.

FIG. 22 shows the distal portion 2005 of the antenna assembly 2014 including the delivery needle 2023 shown in a second configuration in accordance with an embodiment of the present disclosure. Delivery needle 2023 may be formed of any suitable material, and may include one or more portions formed of a flexible material. In some embodiments, as shown in FIG. 22, at least the distal portion of the delivery needle 2023 is formed of a flexible material configured to bend in a curvilinear fashion.

FIGS. 23, 24 and 25 show a portion of an ablation needle 2300 including a tip portion 2323 configured to support a plurality of drug-delivery tines 2385. Referring to FIG. 23, the ablation needle 2300 is shown disposed in a first configuration, wherein the tip portion 2323 has been inserted through tissue "T₁" and positioned into the periphery of the tumor "T₂".

Referring to FIG. 24, the ablation needle 2300 is shown disposed in a second configuration, wherein the tip portion 2323 is disposed within a central region of the tumor "T₂". Referring to FIG. 25, the ablation needle 2300 is shown disposed in a third configuration, wherein the tip portion 2323 is inserted to the distal edge of the tumor "T₂". The drug-delivery tines 2385 may be configured as polymeric controlled-release drug-delivery systems, which may be capable of deployment into tissue, e.g., dislodged and implanted into tissue.

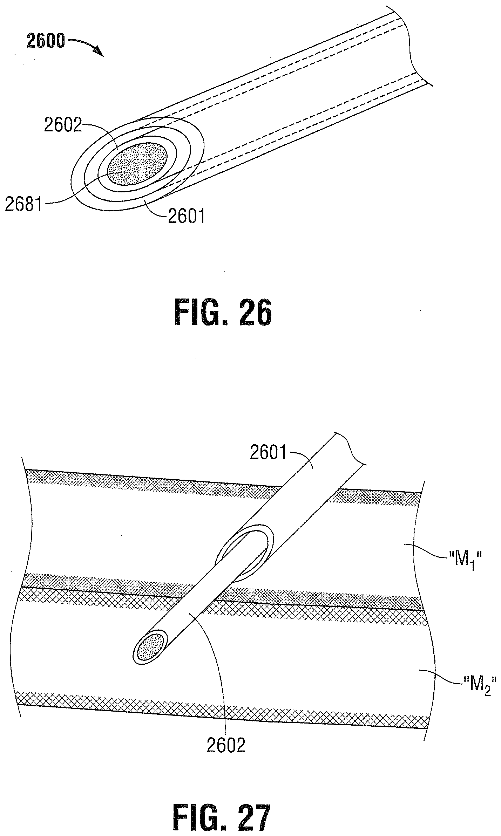

FIGS. 26 and 27 show a portion of an ablation needle 2600 including a delivery needle 2601 in accordance with an embodiment of the present disclosure that includes a micro-needle 2602 adapted to be slideably moveable within the delivery needle 2601. Micro-needle 2602 may be configured to deliver any suitable drug 2681 into a medium. In FIG. 27, the delivery needle 2601 is shown positioned a first medium "M₁", e.g., tissue, with the micro-needle 2602 shown extending distally from the delivery needle 2601 into a second medium "M₂", e.g., tumor.

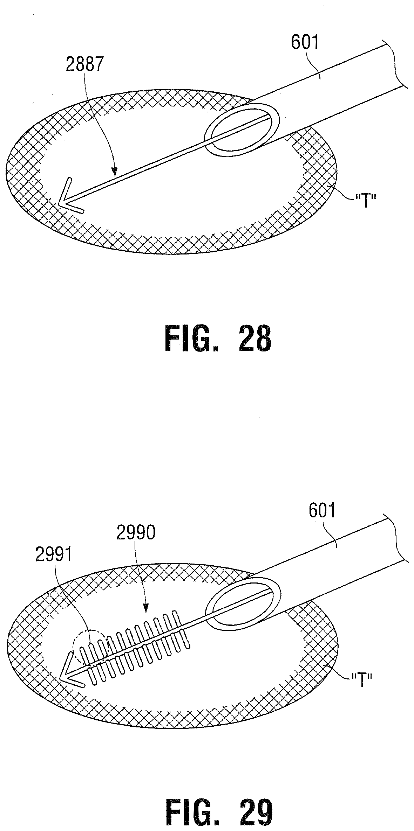

FIG. 28 shows a portion of a delivery needle, such as the delivery needle 601 of the ablation needle 600 shown in FIGS. 6 and 7, shown with a micro-needle 2887 extending distally from the delivery needle 601 into tissue "T".

FIG. 29 shows a portion of a delivery needle, such as the delivery needle 601 of the ablation needle 600 shown in FIGS. 6 and 7, shown with a micro-needle array 2990 including a plurality of micro-needle elements 2991 extending distally from the delivery needle 601 into tissue "T".

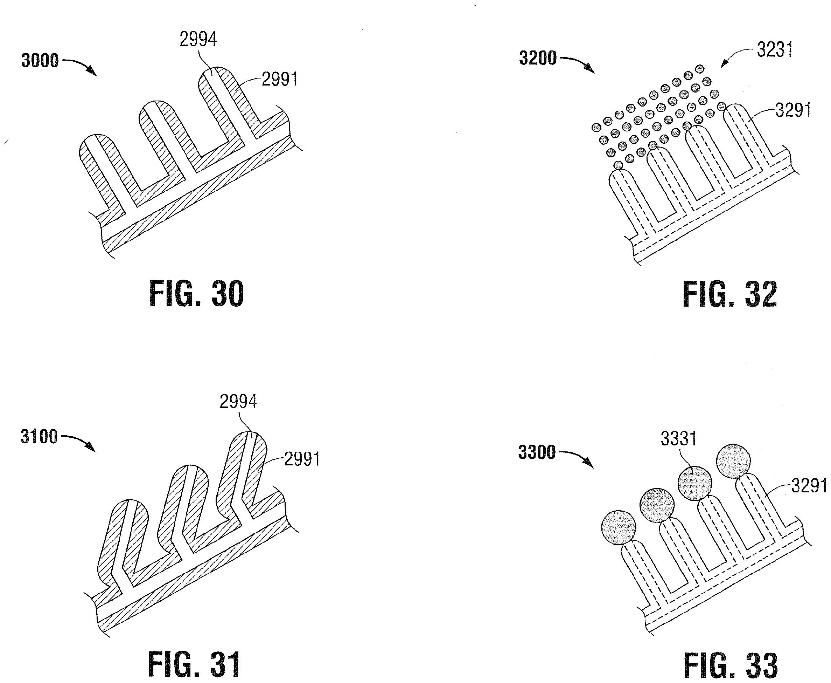

FIG. 30 shows three micro-needle elements 2991 of the micro-needle array 2990 shown in FIG. 29 disposed in a first configuration 3000. FIG. 31 shows a second configuration 3100 of micro-needle elements 2991 of the micro-needle array 2990 of FIG. 29. As seen in FIGS. 30 and 31, each of the micro-needle elements 2991 defines a lumen 2991 therein suitable for delivery of one or more drugs. It is to be understood that the micro-needle elements 2991 may take any of a variety of suitable configurations.

FIG. 32 shows a portion of a micro-needle array 3200 including micro-needle elements 3291 shown with a drug 3231 attached to the micro-needle elements 3291 in accordance with an embodiment of the present disclosure.

FIG. 33 shows a portion of a micro-needle array 3300 including the micro-needle elements 3291 shown in FIG. 32 shown with a drug 3331 attached to the micro-needle elements 3291 in accordance with an embodiment of the present disclosure.

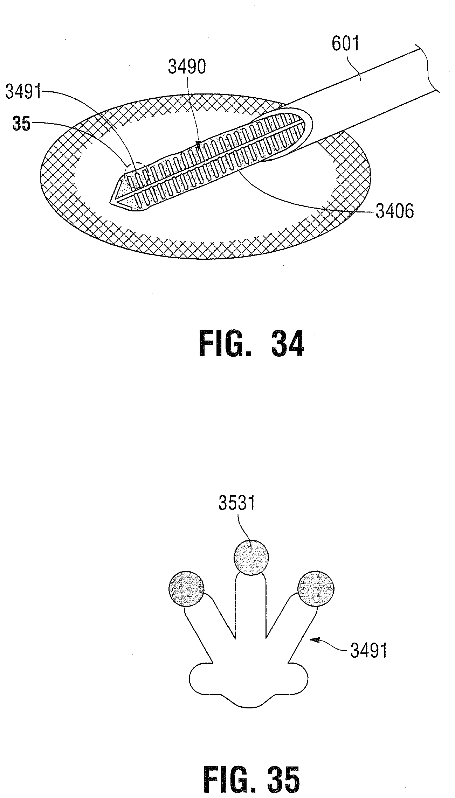

FIG. 34 shows a portion of a delivery needle, such as the delivery needle 601 of the ablation needle 600 shown in FIGS. 6 and 7, shown with a micro-needle array 3490 partially deployed from the delivery needle 601 into tissue "T". Micro-needle array 3490 includes a plurality of micro-needle elements 3491, which may be coated with a heat-sensitive material 3406. FIG. 35 a configuration of the micro-needle elements 3491 with a drug 3531 attached in accordance with an embodiment of the present disclosure.

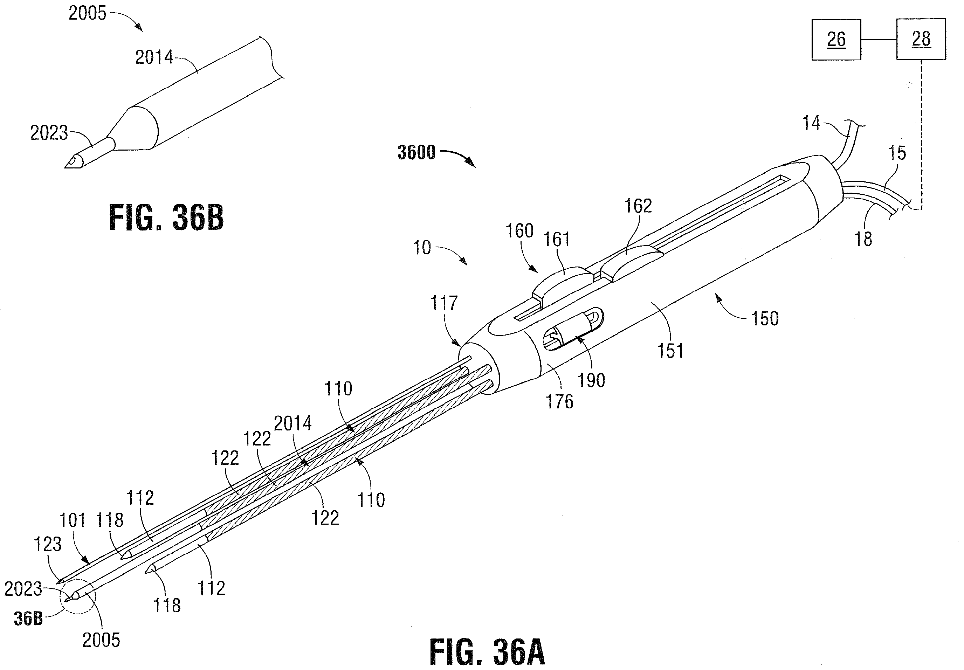

FIG. 36A shows an electrosurgical system (shown generally as 3600) in accordance with an embodiment of the present disclosure that includes an ablation device 10 including a delivery needle 101 and an antenna assembly 2014 including a delivery needle 2023 for use with various surgical procedures. Ablation device 10 generally includes a handle assembly 150 and an array of ablation electrodes 110. Ablation electrodes 110 are operatively connected to an electrosurgical power generating source 28. Ablation device 10 may include additional, fewer, or different components than shown in FIG. 36A, depending upon a particular purpose or to achieve a desired result. In some embodiments, as shown in FIGS. 36A and 36B, the delivery needle 2023 is configured to be slideably moveable within the antenna assembly 2014.

In some embodiments, electrosurgical system 3600 (also referred to herein as ablation system 3600) may include a controller 26 for controlling and/or monitoring the operating parameters of the ablation system 3600. In some embodiments, as shown in FIG. 36A, the controller 26 is communicatively-coupled to the electrosurgical power generating source 28. Controller 26 may additionally, or alternatively, be communicatively-coupled to a fluid source (e.g., coolant source "FS" shown in FIG. 2). Functions of the controller 26 may be integrated with those of the electrosurgical power generating source 28, may be integrated with other components of the electrosurgical system 36000, and/or may be in the form of stand-alone units coupled among components of the electrosurgical system 3600.

Ablation electrode assembly 110 includes an elongated ablation needle 112. In some embodiments, coolant fluid (and/or drug agent) may circulate to a tip portion for cooling of the ablation needle 112. One or more sensors may be utilized to measure temperatures at various locations in the proximity of the tip portion. One or more sensor devices, or components thereof, may be disposed outside the distal end portion 118 of the ablation needle 112. The sensed temperature may be utilized to control the flow of energy and/or the flow of coolant to attain the desired ablation while maintaining the maximum temperature substantially below a predetermined temperature, e.g., 100° C.

In some embodiments, ablation device 10 is adapted to allow the user to selectively position the delivery needle 101 from one or more first configurations, wherein the distal end 123 of the delivery needle 101 is positioned proximal to the distal end portion 118 of the ablation needles 112, to one or more second configurations, wherein at least the distal end 123 of the delivery needle 101 is positioned distally beyond the distal end portion 118 of the ablation needles 112. Ablation device 10 may additionally, or alternatively, be adapted to allow the user to selectively position the antenna assembly 2014 from one or more first configurations, wherein the distal end of the antenna assembly 2014 and/or the delivery needle 2023 is positioned proximal to the distal end portion 118 of the ablation needles 112, to one or more second configurations, wherein at least the distal end of the antenna assembly 2014 and/or the delivery needle 2023 is positioned distally beyond the distal end portion 118 of the ablation needles 112.

Handle assembly 150 generally includes a handle body 151 configured to support the ablation electrodes 110, the delivery needle 101, and the antenna assembly 2014 at the distal end 117 of the handle body 151. Handle assembly 150 includes a slideably moveable member 160 adapted to allow the user to selectively move the delivery needle 101 and/or the antenna assembly 2014 and/or the delivery needle 2023. Slideably moveable member 160 may include one or more buttons having a desired ergonomic form operably associated with the handle body 151. In some embodiments, as shown in FIG. 36A, slideably moveable member 160 includes a first member 161, e.g., adapted to allow the user to selectively move the delivery needle 101, and a second member 162, e.g., adapted to allow the user to selectively move the antenna assembly 2014, wherein the first member 161 and the second members 162 are independently, slideably moveable. In some embodiments, slideably moveable member 160 may additionally, or alternatively, be configured to allow the user to selectively initiate/activate the delivery of drug and/or contrast agent from the supply line 14 to the delivery needle 101. Handle assembly 150 may additionally, or alternatively, include a slideably moveable member 190, e.g., thumb-slide actuator, adapted to allow the user to selectively position the delivery needle 2023 from one or more first configurations, wherein the delivery needle 2023 is positioned within the antenna assembly 2014, to one or more second configurations, wherein the delivery needle 2023, or portion thereof, is positioned distally beyond the distal end of the antenna assembly 2014 (FIG. 36B).

In some embodiments, transmission line 15 may provide a conduit (not shown) configured to provide coolant from a coolant source, e.g., deionized water, or other suitable cooling medium, for cooling one or more components of the ablation device 10, such as the ablation electrodes 110. Transmission line 15 may additionally, or alternatively, provide a conduit (not shown) configured to provide drugs and/or contrast agent to the handle assembly 150 and/or the delivery needle 101 and/or the delivery needle 2023.

A drug and/or contrast agent supply line 14 may be provided to fluidly-couple the ablation device 10 to a source of the drug and/or contrast agent delivery supply for supplying drugs and/or contrast agent to the handle assembly 150 and/or the delivery needle 101 and/or the delivery needle 2023. Handle assembly 150 may include one or more fluid conduits (not shown) associated with the handle body 151 configured to provide fluid communication between the supply line 14 and the delivery needle 101 and/or the delivery needle 2023.

During ablation, e.g., using the electrosurgical system 3600, the ablation electrodes 110 and the antenna assembly 2014 are inserted into or placed into the body of a patient, e.g., percutaneously or intraoperatively. Ultrasound or computed tomography (CT) guidance may be used to accurately guide the ablation electrodes 110 and the antenna assembly 2014 into the area of tissue to be treated. Electrosurgical power generating source 28 may be the source of high-frequency voltage which produces the high-frequency current that emanates from the distal end portion 118 of ablation needles 112. Following treatment or ablation of the target tissue, ablation electrodes 110 and the antenna assembly 2014 may be withdrawn from the target site and introduced into another target site, into the same target site from a different angle or approach, or in substantially the same location.

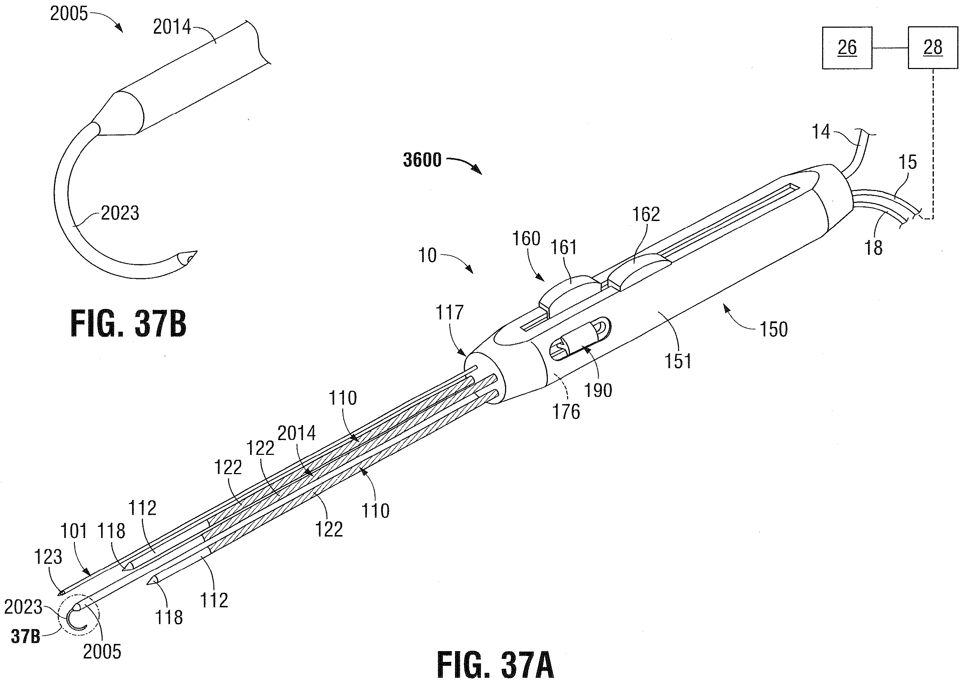

FIG. 37A shows the electrosurgical system 3600 of FIG. 36A, wherein at least a portion of the delivery needle 2023 of the antenna assembly 2014 is formed of a flexible material. In some embodiments, as shown in FIG 22 and FIGS. 37A and 37B, at least the distal portion of the delivery needle 2023 is formed of a flexible material configured to bend in a curvilinear fashion. Controlled delivery of a drug agent from an implantable formulation over periods of time (e.g., periods of time measured in days or weeks) may help to assure patient compliance, as implantable formulations are not easily tampered with by the patient and can be designed to provide therapeutic doses of beneficial agent over periods of days or weeks without patient input. In accordance with embodiments of the present disclosure wherein an implantable formulation may be placed during an ablation procedure, possible concerns over intrusive access may be markedly mitigated. Other potential benefits of the use of implantable formulation placed during an ablation procedure according to embodiments of the present disclosure include non-permanent functional life obviating extraction, reduced site irritation, fewer occupational hazards for patients and practitioners, reduced waste disposal hazards, decreased costs, and increased efficacy as compared to other parenteral administration techniques, such as injections, which may require multiple administrations over relatively short time intervals.

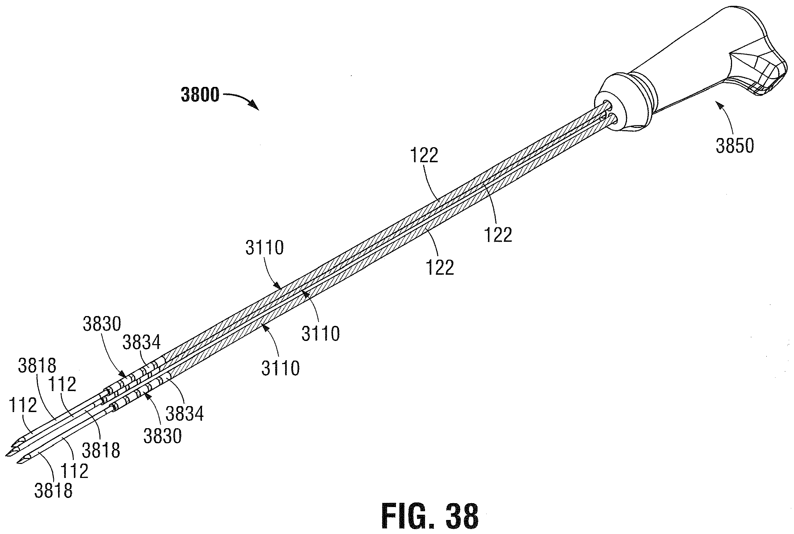

FIG. 38 shows an ablation device 3800 in accordance with the invention that includes a plurality of the ablation electrodes 3110 including a plurality of drug reservoir divots associated therewith. Any suitable number of the same or different drug reservoir divots may be utilized. Ablation device 3800 includes a handle assembly 3850. In some embodiments, as shown in FIG. 38, the ablation device 3800 includes three ablation electrodes 3110 supported on and/or operatively connected to the handle assembly 3850. The shape, size, and number of the electrodes 3110 may be varied from the configuration depicted in FIG. 38. Ablation device 3800 may include any feature or combination of features of the ablation device embodiments disclosed herein.

As shown in FIG. 38, each ablation electrode 3110 includes an ablation needle 112. Ablation electrodes 3110 include an insulative coating 122 over at least a portion of the length of the ablation needle 112 thereof. In some embodiments, the insulative coating 122 is disposed over substantially the length of the ablation needle 112. In some embodiments, as shown in FIG. 38, the insulative coating 122 extends toward the distal end of the ablation needle 112, such that the distal end portion 3818 of the ablation needle 112 is exposed or non-insulated.

A plurality of recesses 3830 is defined in the distal end portion of the insulative coating 122, e.g., proximate to the distal end portion 3818 of the ablation needle 112. The recesses 3830 may be formed in any suitable shape, and may define receptacles of any suitable volume to contain one or more drugs. In some embodiments, as shown in FIG. 38, the recesses 3830 are configured as annular grooves disposed surrounding a portion of the ablation needles 112. The number, size, and position of the recesses 3830 may be varied from the configuration depicted in FIG. 38.

One or more of the recesses 3830 are provided with one or more drugs 3834 therein, such as without limitation, microspheres, chemotherapeutic agents, and/or a thermo-sensitive binding agent, e.g., wax. In some embodiments, the recesses 3830 are provided with one or more chemotherapeutic agents, and may be provided with a thermo-sensitive binding agent. A thermo-sensitive binding agent may be combined with the microspheres, API, or CTA disposed in the recesses 3830. A thermo-sensitive binding agent may additionally, or alternatively, be formed as layered coating to protect and/or postpone delivery of the microspheres, API, or CTA.

A variety of drug agents may be delivered by devices according to embodiments of the present disclosure. Some examples of drug agents which may be delivered by devices according to embodiments of the present disclosure include chemotherapeutic agents such as without limitation cisplatin, paclitaxel, doxorubicin, fluorouracil, as well as other compounds such as without limitation prochlorperzine edisylate, ferrous sulfate, aminocaproic acid, mecamylamine hydrochloride, procainamide hydrochloride, amphetamine sulfate, methamphetamine hydrochloride, benzamphetamine hydrochloride, isoproterenol sulfate, phenmetrazine hydrochloride, bethanechol chloride, methacholine chloride, pilocarpine hydrochloride, atropine sulfate, scopolamine bromide, isopropaniide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperzine maleate, anisindone, diphenadione erythrityl tetranitrate, digoxin, isofluorophate, acetazolamide, methazolamide, bendroflumethiazide, chloropromaide, tolazamide, chlormadinone acetate, phenaglycodol, allopurinol, aluminum aspirin, methotrexate, acetyl sulfisoxazole, erythromycin, hydrocortisone, hydrocorticosterone acetate, cortisone acetate, dexamethasone and its derivatives such as betamethasone, triamcinolone, methyltestosterone, 17-S-estradiol, ethinyl estradiol, ethinyl estradiol 3-methyl ether, prednisolone, 17-oc-hydroxyprogesterone acetate, 19-nor-progesterone, norgestrel, norethindrone, norethisterone, norethiederone, progesterone, norgesterone, norethynodrel, aspirin, indornethacin, naproxen, fenoprofen, sulindac, indoprofen, nitroglycerin, isosorbide dinitrate, propranolol, timolol, atenolol, aiprenolol, cimetidine, clonidine, imipramine, levodopa, chlorpromazine, methyldopa, dihydroxyphenylalanine, theophylline, calcium gluconate, ketoprofen, ibuprofen, cephalexin, erythromycin, haloperidol, zomepirac, ferrous lactate, vincamine, diazepam, phenoxybenzamine, diltiazem, mitrinone, capropril, mandol, quanbenz, hydrochlorothiazide, ranitidine, flurbiprofen, fenufen, fluprofen, tolmetin, alciofenac, mefenamic, flufenamic, difiuinal, nimodipine, nitrendipine, nisoldipine, nicardipine, felodipine, lidoflazine, tiapamil, gallopamul, amlodipine, mioflazine, lisinoipril, enalapril, enalaprilat, captopril, ramipril, famotidine, nizatidine, sucralfate, etintidine, tetratolol, minoxidil, chlordazepoxide, diazepam, amitriptyline, and imipramine; opioids such as meperidine, hydrocodone, oxycodone, and semi-synthetic opioids such as oxymorphone, hydromorphone, opiates such as morphine and codeine, opioid antagonists such as without limitation naltrexone, nalbuphine, naloxone as well as opioid agonist/antagonist compounds such as buprenorphine, and synthetic analgesics such as methadone, tramadol, fentanyl and sufentanil.

Some other examples of drug agents which may be delivered by devices according to embodiments of the present disclosure include vitamin and supplements such as vitamins B-12 (cyanocobalamin) and D2, anti-virals such as without limitation acyclorvir and zidovudine; proteins and peptides such as without limitation insulin, colchicine, glucagon, thyroid stimulating hormone, parathyroid and pituitary hormones, calcitonin, renin, prolactin, corticotrdphin, thyrotropic hormone, follicle stimulating hormone, chorionic gonadotropin, gonadotropin releasing hormone, bovine somatotropin, porcine somatotropin, oxytocin, vasopressin, GRE, prolactin, somatostatin, lypressin, pancreozymin, luteinizing hormone, LHRH, LHRH agonists and antagonists, leuprolide, interferons, interleukins, growth hormones such as human growth hormone, bovine growth hormone and porcine growth hormone, fertility inhibitors such as the prostaglandins, fertility promoters, growth factors, coagulation factors, human pancreas hormone releasing factor, analogs and derivatives of these compounds, and pharmaceutically acceptable salts of these compounds, or their analogs or derivatives. On the molecular level, the various forms of the beneficial agent may include uncharged molecules, molecular complexes, and pharmaceutically acceptable acid addition and base addition salts such as hydrochlorides, hydrobromides, acetate, sulfate, laurylate, oleate, and salicylate. Examples of acidic compounds which may be delivered by devices according to embodiments of the present disclosure include salts of metals, amines or organic cations. Derivatives such as esters, ethers and amides may also be used.

A drug agent for delivery by devices according to embodiments of the present disclosure may be used alone or mixed with other agents. A drug agent for delivery by the presently-disclosed devices may include pharmaceutically acceptable excipients, polymeric carriers and/or additional ingredients, such as antioxidants, stabilizing agents, permeation enhancers, polysaccharides, proteins, nucleotides like aptamers, and fatty acids, etc., and fabricated into different forms, such as solution, suspension, gel, colloidal dispersion like liposome, or micro- and nano-particles for controlled delivery of the drug agent. A drug agent for delivery by the presently-disclosed devices may include a thermo-sensitive metal depositor or any such compound that increases the sensitivity of the target tissue, e.g., tumor, to ablation.

A drug agent for delivery by the presently-disclosed devices may include a cryoablation agent, e.g., liquid nitrogen, and may prove complementary to thermal ablation that uses electrosurgical energy at RF or microwave frequencies.

The above-described systems and ablation devices may offer improved anticancer efficacy with RF ablation (or microwave ablation) and localized drug delivery capabilities integrated into a single medical device. In accordance with the above-described systems and ablation devices, an approach is taken to deliver drug formulation(s) locally when the anatomical access has already been obtained for the purpose of RF or microwave ablation, which, in turn, presents the prospect of reduced side-effects associated with systemic administration of the same drug molecule(s).

In accordance with the above-described systems and ablation devices, heat activated drugs may be delivered to the periphery of the tumor, which may not get as hot as the center of the tumor, to ensure adequate margins. The above-described systems and ablation devices may be used to kill tumors from the inside out, wherein the temperature at the periphery may not be high enough to destroy the tumor through ablation (e.g., in some cases, requiring temperatures of at least 55° C), but at high enough temperature (e.g., in some cases, temperatures of about 45° C) to activate one or more drugs delivered by the above-described ablation devices, which may take care of killing the tumor edges.

Although exemplary embodiments have been described in detail with reference to the accompanying drawings for the purpose of illustration and description, it is to be understood that the inventive processes and apparatus are not to be construed as limited thereby.

## Claims

1. An ablation device (3800), comprising:
a handle assembly (3850); and
an array of ablation electrodes (3110) operably associated with the handle assembly, wherein at least one ablation electrode of the array of ablation electrodes includes a recess (3830) defined therein, and wherein a drug is disposed at least in part within the recess, wherein the at least one ablation electrode includes an ablation needle (112), and wherein the at least one ablation electrode includes an insulative coating (122) over at least a portion of the length of the ablation needle thereof, **characterised in that** at least one recess (3830) is defined in a distal end portion of the insulative coating.

2. The ablation device of claim 1, further comprising an antenna assembly extending from the handle assembly, wherein the antenna assembly includes a delivery needle.

3. The ablation device of claim 1, wherein the insulative coating is disposed over substantially the length of the at least one ablation needle.

4. The ablation device of claim 1 or 3, wherein the insulative coating extends toward a distal end of the at least one ablation needle, such that a distal end portion of the at least one ablation needle is exposed or non-insulated.

5. The ablation device of claim 4, wherein the at least one recess is proximate to the distal end portion of the ablation needle.

6. The ablation device of claim 1, 3, 4 or 5, wherein the at least one recess is configured as an annular groove disposed surrounding a portion of the at lest one ablation needle.

7. The ablation device of any one of the preceding claims, comprising a drug and a thermo-sensitive binding agent disposed in the at least one recess,

8. The ablation device of any preceding claim, wherein the drug comprises microspheres, and/or chemotherapeutic agents.

9. The ablation device of any one of the preceding claims, wherein the drug includes microspheres and a thermo-sensitive binding agent is combined with the microspheres and/or a thermosensitive binding agent is formed as a layered coating to protect and/or postpone delivery of microspheres included in the drug.

## Patentansprüche

1. Ablationsvorrichtung (3800), umfassend:
eine Handgriffbaugruppe (3850); und
eine Anordnung von Ablationselektroden (3110), die mit der Handgriffbaugruppe betriebsmäßig verbunden sind, wobei zumindest eine Ablationselektrode der Anordnung von Ablationselektroden eine darin definierte Aussparung (3830) aufweist und wobei ein Arzneimittel zumindest teilweise innerhalb der Aussparung angeordnet ist, wobei die zumindest eine Ablationselektrode eine Ablationsnadel (112) aufweist und wobei die zumindest eine Ablationselektrode eine isolierende Beschichtung (122) über zumindest einem Abschnitt der Länge der Ablationsnadel davon aufweist, **dadurch gekennzeichnet, dass** zumindest eine Aussparung (3830) in einem distalen Endabschnitt der isolierenden Beschichtung definiert ist.

2. Ablationsvorrichtung nach Anspruch 1, wobei umfassend eine Antennenanordnung, die sich von der Handgriffbaugruppe erstreckt, wobei die Antennenanordnung eine Abgabenadel aufweist.

3. Ablationsvorrichtung nach Anspruch 1, wobei die isolierende Beschichtung über im Wesentlichen der Länge der zumindest einen Ablationsnadel angeordnet ist.

4. Ablationsvorrichtung nach Anspruch 1 oder 3, wobei sich die isolierende Beschichtung in Richtung eines distalen Endes der zumindest einen Ablationsnadel erstreckt, so dass ein distaler Endabschnitt der zumindest einen Ablationsnadel freiliegt oder nicht isoliert ist.

5. Ablationsvorrichtung nach Anspruch 4, wobei sich die zumindest eine Aussparung in der Nähe von dem distalen Endabschnitt der Ablationsnadel befindet.

6. Ablationsvorrichtung nach Anspruch 1, 3, 4 oder 5, wobei die zumindest eine Aussparung als eine ringförmige Rille konfiguriert ist, die einen Abschnitt der zumindest einen Ablationsnadel umgebend angeordnet ist.

7. Ablationsvorrichtung nach einem vorstehenden Anspruch, umfassend ein Arzneimittel und ein wärmeempfindliches Bindemittel, das in der zumindest einen Aussparung angeordnet ist.

8. Ablationsvorrichtung nach einem vorstehenden Anspruch, wobei das Arzneimittel Mikrosphären und/oder chemotherapeutische Wirkstoffe umfasst.

9. Ablationsvorrichtung nach einem der vorstehenden Ansprüche, wobei das Arzneimittel Mikrosphären aufweist und ein wärmeempfindliches Bindemittel mit den Mikrosphären kombiniert ist und/oder ein wärmeempfindliches Bindemittel als eine geschichtete Beschichtung ausgebildet ist, um eine Abgabe von in dem Arzneimittel enthaltenen Mikrosphären zu schützen und/oder zu verschieben.

## Revendications

1. Dispositif d'ablation (3800), comprenant :
un ensemble de poignée (3850) ; et
un agencement d'électrodes d'ablation (3110) associé en fonctionnement à l'ensemble de poignée, dans lequel au moins une électrode d'ablation de l'agencement d'électrodes d'ablation inclut un évidement (3830) défini dedans, et dans lequel un médicament est disposé au moins en partie dans l'évidement, dans lequel l'au moins une électrode d'ablation inclut une aiguille d'ablation (112), et dans lequel l'au moins une électrode d'ablation inclut un revêtement isolant (122) sur au moins une portion de la longueur de l'aiguille d'ablation de celle-ci, **caractérisé en ce que**
au moins un évidement (3830) est défini dans une portion d'extrémité distale du revêtement isolant.

2. Dispositif d'ablation selon la revendication 1, comprenant en outre un ensemble d'antenne s'étendant depuis l'ensemble de poignée, dans lequel l'ensemble d'antenne inclut une aiguille d'administration.

3. Dispositif d'ablation selon la revendication 1, dans lequel le revêtement isolant est disposé sur sensiblement la longueur de l'au moins une aiguille d'ablation.

4. Dispositif d'ablation selon la revendication 1 ou 3, dans lequel le revêtement isolant s'étend vers une extrémité distale de l'au moins une aiguille d'ablation de sorte qu'une portion d'extrémité distale de l'au moins une aiguille d'ablation soit exposée ou non isolée.

5. Dispositif d'ablation selon la revendication 4, dans lequel l'au moins un évidement est à proximité de la portion d'extrémité distale de l'aiguille d'ablation.

6. Dispositif d'ablation selon la revendication 1, 3, 4 ou 5, dans lequel l'au moins un évidement est configuré en tant que rainure annulaire disposée entourant une portion de l'au moins une aiguille d'ablation.

7. Dispositif d'ablation selon l'une quelconque des revendications précédentes, comprenant un médicament et un agent de liaison thermosensible disposé dans l'au moins un évidement.

8. Dispositif d'ablation selon l'une quelconque des revendications précédentes, dans lequel le médicament comprend des microsphères, et/ou des agents chimiothérapeutiques.

9. Dispositif d'ablation selon l'une quelconque des revendications précédentes, dans lequel le médicament inclut des microsphères et un agent de liaison thermosensible est combiné avec les microsphères et/ou un agent de liaison thermosensible est formé en tant que revêtement à couches pour protéger et/ou reporter l'administration de microsphères incluses dans le médicament.
